# EUROPEAN PATENT SPECIFICATION

(11) **EP 1 088 066 B1**
(45) Date of publication and mention of the grant of the patent: **29.11.2006**
(21) Application number: 99928945.7
(22) Date of filing: 17.06.1999
(51) Int. Cl.: C12N 15/11, C07H 21/00, A61K 31/70, C07H 19/09, C07H 19/19

(54) **ANTISENSE OLIGONUCLEOTIDE CONSTRUCTS BASED ON BETA-ARABINOFURANOSE AND ITS ANALOGUES**
AUF BETA-ARABINOSE, UND DESSEN ANALOGEN, BASIERTE ANTISENSE OLIGONUKLEOTIDE
CONSTRUCTIONS OLIGONUCLEOTIDES ANTISENSE A BASE DE BETA-ARABINOFURANOSE ET DE SES ANALOGUES

(30) Priority: 19.06.1998 CA 2241361
(43) Date of publication of application: 04.04.2001
(73) Proprietor: McGill University, Montreal, Québec H3A 2T5 (CA)
(72) Inventor: DAMHA, Massad, José, St.-Hubert, Québec J3Y 8N9 (CA); PARNIAK, Michael, A., Verdun, Québec H4H 1S7 (CA); NORONHA, Anne M., Montréal, Québec H2K 2V2 (CA); WILDS, Christopher, Pincourt, Québec G2E 1X3 (CA); BORKOW, Gadi, 76910 Kfar Gibton, Rehovot (IL); ARION, Dominique, Montréal, Québec H3V 1B4 (CA)
(74) Representative: Nargolwalla, Cyra
(86) International application number: PCT/CA1999/000571
(87) International publication number: WO 1999/067378

(56) References cited:
- WO-A-90/03370
- WO-A-93/10820
- NORONHA A. ET AL.: "Triple helices containing arabinonucleotides in the third (Hoogsteen) strand: effects of inverted stereochemistry at the 2'-position of the sugar moiety." NUCLEIC ACIDS RES 1998 JUN 1;26(11):2665-71, XP002119321 cited in the application
- AOYAGI, M. ET AL.: "Effects of 2'-alpha- and 2'-beta-bromo-2'-deoxyadenosine on oligonucleotide hybridization and nuclease stability." BIOORGANIC & MEDICINAL CHEMISTRY LETTERS, vol. 6, 1996, pages 1573-1576, XP004175756 ISSN: 0960-894X
- GIANNARIS P.A. ET AL: "Hybridization properties of oligoarabinonucleotides." CANADIAN JOURNAL OF CHEMISTRY, (1994) 72/3 (909-918), XP002119322 cited in the application
- SANGHVI, Y. & COOK, D.: "Carbohydrates: synthetic methods and applications in antisense therapeutics" ACS SYMPOSIUM SERIES, vol. 580, 13 March 1994 (1994-03-13), pages 1-22, XP002119323 ISSN: 0097-6156 cited in the application
- ALTMANN, K.-H. ET AL.: "Novel chemistry" STEIN, C.A. & KRIEG, A.M. 'APPLIED ANTISENSE OLIGONUCLEOTIDE TECHNOLOGY'. WILEY-LISS, NEW YORK, US;1998, pages 73-107, XP002119324
- NORONHA, A. & DAMHA, M.: "Hybridization properties of arabinonucleic acids (ANA), influence of stereochemistry at 2' on the stability of double and triple helices" JOURNAL OF BIOMOLECULAR STRUCTURE & DYNAMICS, vol. 14, June 1997 (1997-06), pages 805-806, XP002119325
- XODO LE. ET AL.: "Effect of 5-methylcytosine on the structure and stability of DNA. Formation of triple-stranded concatenamers by overlapping oligonucleotides." J BIOMOL STRUCT DYN 1994 FEB;11(4):703-20, XP002119326
- FLANAGAN, M. ET AL.: "Effects of oligonucleotide length, mismatches and mRNA levels on C-5 propyne modified antisense potency" NUCLEIC ACIDS RESEARCH., vol. 24, 1996, pages 2936-2941, XP002119327 ISSN: 0305-1048 cited in the application
- WILDS CJ. ET AL.: "Duplex recognition by oligonucleotides containing 2'-deoxy-2'-fluoro-D-arabinose and 2'-deoxy-2'-fluoro-D-ribose. Intermolecular 2'-OH-phosphate contacts versus sugar puckering in the stabilization of triple-helical complexes." BIOCONJUG CHEM 1999 MAR-APR;10(2):299-305, XP002119328
- DAMHA M.J. ET AL: "Hybrids of RNA and arabinonucleic acids (ANA and 2'F-ANA) are substrates of ribonuclease H." JOURNAL OF THE AMERICAN CHEMICAL SOCIETY, (16 DEC 1998) 120/49 (12976-12977)., XP002119329

### SEQUENCE LISTING

<110> McGILL UNIVERSITY
   DAMHA, Massad, José
   PARNIAK, Michael, A.
   NORONHA, Anne, M.
   WILDS, Christopher
   BORKOW, Gadi
   ARION, Dominique
<120> ANTISENSE OLIGONUCLEOTIDE CONSTRUCTS BASED ON BETA-ARABINOFURANOSE AND ITS ANALOGUES
<130> 1770-206"PCT" FC/ld
<150> CA 2,241,361
<151> 1998-06-19
<160> 17
<170> FastSEQ for Windows Version 3.0
<210> 1
<211> 18
<212> RNA
<213> Artificial Sequence
<220>
<223> Use as an oligomer
<400> 1
   agcucccagg cucagauc 18
<210> 2
<211> 18
<212> DNA
<213> Artificial Sequence
<220>
<223> Use as an oligomer
<400> 2
   aaaaaaaaaa aaaaaaaa 18
<210> 3
<211> 18
<212> RNA
<213> Artificial Sequence
<220>
<223> Use as an oligomer
<400> 3
   uuuuuuuuuu uuuuuuuu 18
<210> 4
<211> 18
<212> RNA
<213> Artificial Sequence
<220>
<223> Use as an oligomer
<400> 4
   uuauauuuuu ucuuuccc 18
<210> 5
<211> 18
<212> RNA
<213> Artificial Sequence
<220>
<223> Use as an oligomer
<400> 5
   auauccuugu cguauccc 18
<210> 6
<211> 18
<212> DNA
<213> Artificial Sequence
<220>
<223> Use as an oligomer
<400> 6
   agctcccagg ctcagatc 18
<210> 7
<211> 18
<212> DNA
<213> Artificial Sequence
<220>
<223> Use as an oligomer
<400> 7
   tttttttttt tttttttt 18
<210> 8
<211> 18
<212> DNA
<213> Artificial Sequence
<220>
<223> Use as an oligomer
<400> 8
   aaaaaaaaaa aaaaaaaa 18
<210> 9
<211> 18
<212> DNA
<213> Artificial Sequence
<220>
<223> Use as an oligomer
<400> 9
   ttatattttt tctttccc 18
<210> 10
<211> 18
<212> DNA
<213> Artificial Sequence
<220>
<223> Use as an oligomer
<400> 10
   atatccttgt cgtatccc 18
<210> 11
<211> 28
<212> DNA
<213> Artificial Sequence
<220>
<223> Use as an oligomer
<400> 11
   ggagaggagg gatttttccc tcctctcc 28
<210> 12
<211> 28
<212> RNA
<213> Artificial Sequence
<220>
<223> Use as an oligomer
<400> 12
   ggagaggagg gattttuccc uccucucc 28
<210> 13
<211> 12
<212> DNA
<213> Artificial Sequence
<220>
<223> Use as an oligomer
<400> 13
   cctctcctcc ct 12
<210> 14
<211> 18
<212> DNA
<213> Artificial Sequence
<220>
<223> Use as an oligomer
<400> 14
   agctcccagg ctcagatc 18
<210> 15
<211> 18
<212> RNA
<213> Artificial Sequence
<220>
<223> Use as an oligomer
<400> 15
   agcucccagg cucagauc 18
<210> 16
<211> 18
<212> RNA
<213> Artificial Sequence
<220>
<223> Use as an oligomer
<400> 16
   agcucccagg cucagauc 18
<210> 17
<211> 18
<212> DNA
<213> Artificial Sequence
<220>
<223> Use as an oligomer
<400> 17
   taatccctat cgtcgctt 18

## Description

### BACKGROUND OF THE INVENTION

### (a) Field of the Invention

It is the primary objective of this invention to provide modified oligonucleotide therapeutic agents to selectively prevent gene transcription and expression in a sequence-specific manner. In particular, this invention is directed to the selective inhibition of protein biosynthesis via antisense strategy using oligonucleotides constructed from arabinonucleotide or modified arabinonucleotide residues. More particularly this invention relates to the use of antisense oligonucleotides having arabinose sugars to hybridize to complementary RNA such as cellular messenger RNA, viral RNA, etc. More particularly this invention relates to the use of arabinonucleic acid or modified arabinonucleic acid strands to hybridize to and induce cleavage of (via RNaseH activation) the complementary RNA. Other applications of this invention relates to the use of antisense oligonucleotides based on arabinonucleotides or modified arabinonucleotides in combination with RNaseH as laboratory reagents for the sequence specific cleavage and mapping of RNA. This invention also relates to the use of oligonucleotides based on arabinonucleotides or modified arabinonucleotides, particularly those comprised of 2'F-arabinonucleic acid strands to hybridize duplex DNA to form a triple helical complex and thereby block DNA transcription.

### (b) Description of Prior Art

### The Antisense Strategy

Antisense oligonucleotides (AON) are novel therapeutic agents which can inhibit specific gene expression in a sequence-specific manner. Many AON are currently in clinical-trial for the treatment of cancer and viral diseases (for reviews see: (i) Uhlmann, E.; Peyman, A. *Chem. Rev.* 1990, *90*, 543. (ii) Cook, P.D. *Anti-Cancer Drug Design* 1991, *6*, 585. (iii) Crooke, S.T. *Annu. Rev*. *Pharmacol. Toxicol.* 1992, *32*, 329. (iv) Crooke, S.T.; Lebleu, B. *Antisense Research and Applications;* 1993, pp.579, CRC Press, Boca Raton, FL. (v) Agrawal, S.; Iyer, R.P. *Cur. Op. Biotech.* 1995, *6,* 12.). (vi) DeMesmaeker, A.; Haner, R.; Martin, P.; Moser, H. *Acc. Chem. Res*. 1995, *28*, 366. (vii) Crooke, S.T.; Bennett, C.F. *Annu. Rev*. *Pharmacol. Toxicol.* 1996, 36, 107). For potential clinical utility, AON should exhibit stability against degradation by serum and cellular nucleases, show low non-specific binding to serum and cell proteins (this binding would diminish the amount of antisense oligonucleotide available to base-pair with the target RNA), exhibit enhanced recognition of the target RNA sequence (in other words, provide increased stability of the antisense-target RNA duplex at physiological temperature), and to some extent, demonstrate cell-membrane permeability. The formation of a duplex between the antisense oligomer and its target RNA blocks the translation of that RNA, by a mechanism termed "translation arrest". This mechanism may however be a minor contributor to the overall antisense effect. More important is the ability of the antisense oligonucleotide to induce the activation of ribonuclease H (RNaseH), an endogenous enzyme that specifically degrades RNA when duplexed with a complementary DNA oligonucleotide (or antisense oligonucleotide) component (Walder, R.T.; Walder, J.A. *Proc. Natl. Acad. Sci. USA* 1988, *85,* 5011). For example, when an antisense DNA oligonucleotide hybridizes to a mRNA transcript, RNase H then cuts the mRNA at that site. Antisense oligomers that modulate gene expression by more than one mechanism of action are highly desirable as this increases the potential efficacy of the antisense compound *in vivo.*

### Oligonucleotide Analogs

Oligonucleotides containing natural sugars (D-ribose and D-2-deoxyribose) and phosphodiester (PO) linkages are rapidly degraded by serum and intracellular nucleases, which limits their utility as effective therapeutic agents. Chemical strategies to improve nuclease stability include modification of the sugar moiety, the base moiety, and/or modification or replacement of the internucleotide phosphodiester linkage. To date, the most widely studied analogues are the phosphorothioate (PS) oligodeoxynucleotides, in which one of the non-bridging oxygen atoms in the phosphodiester backbone is replaced with a sulfur (Eckstein, F. *Ann. Rev. Biochem.* 1985, *54,* 367).

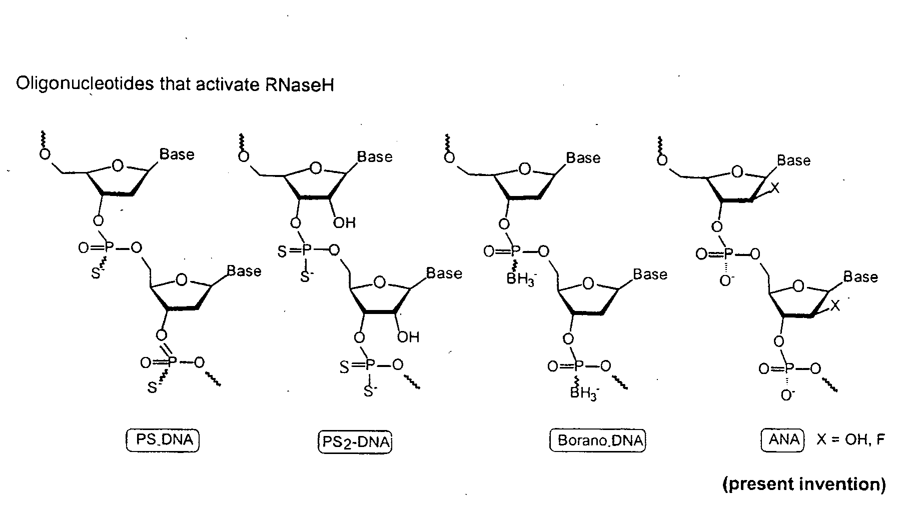

Several phosphorothioate oligonucleotide analogues are undergoing clinical trial evaluation in the treatment of cancer and viral diseases, and some are moving rapidly towards New Drug Application (NDA) filings (Akhtar, S.; Agrawal, S. *"In vivo* studies with *antisense oligonucleotides" TiPS* 1997, *18,* 12). Phosphorothioates retain the ability to induce RNaseH degradation of the target RNA and exhibit good stability to degradation by nucleases. However, the PS oligodeoxynucleotides form less stable duplexes with the target nucleic acid than do PO oligodeoxynucleotides, and also exhibit significant nonspecific binding to cellular proteins, which can reduce the probability of finding and interacting with the target nucleic acid; these characteristics can limit the therapeutic utility of PS-AON (for a review see: Brach, A.D.; "A good *antisense molecule is hard to find", TIBS,* 1998, *23,* 45). Furthermore, PS-AONs are less efficient at inducing RNaseH degradation of the target RNA than are the corresponding PO-AONs (Agrawal, S.; Mayrand, S.H.; Zamenick, P.; Pederson, T. *Proc. Natl. Acad. Sci. USA* 1990, *87,* 1401).

Specificity of action may be improved by developing novel oligonucleotide analogues. Current strategies to generate novel oligonucleotides are to alter the internucleotide phosphate backbone, the heterocyclic base, and the sugar ring, or a combination of these. Alteration or complete replacement of the internucleotide linkage has been the most popular approach, with over 60 types of modified phosphate backbones studied since 1994 (Sanghvi, Y. DNA in "Altered Backbones in Antisense Applications", in *Comprehensive Natural Product Chemistry,* Barton, D.H.R.; Nakanishi, K.; Meth-Coth, O. (eds), 1998, Elsevier Science, Oxford, UK). Apart from the phosphorothioate backbone, only two others have been reported to activate RNaseH activity, i.e., the phosphorodithioate (PS₂) (Seeberger, P.H.; Yen, E.; Caruthers, M.H. *J. Am. Chem. Soc.* 1995, *117,* 1472) and the boranophosphonate backbones (Sergueev, D. et al., Poster 269, XIII International Round Table, Montpellier, France, Sept. 6-10, 1998; Higson, A.P. *et al. Tetrahedron Letters* 1998, *39,* 3899). Because of the higher sulfur content of phosphorodithioate-linked (PS₂) oligodeoxynucleotides, they appear to bind proteins tighter than the phosphorothioate (PS) oligomers, and to activate RNaseH mediated cleavage with reduced efficiency compared to the PS analogue. Boranophosphonate-linked oligodeoxynucleotides activate RNaseH mediated cleavage of RNA targets, but less well than PO- or PS-linked oligodeoxynucleotides.

Among the reported sugar-modified oligonucleotides most of them contain a five-membered ring, closely resembling the sugar of DNA (D-2-deoxyribose) and RNA (D-ribose). Example of these are α-oligodeoxynucleotide analogs, wherein the configuration of the 1' (or anomeric) carbon has been inverted as shown below (Morvan, F.; Rayner, B.; Imbach, J.-L., Chang, D.K.; Lown, J.W. *Nucleic Acids Res.* 1987, *15,* 7027).

These analogues are nuclease resistant, form stable duplexes with DNA and RNA sequences, and are capable of inhibiting β-globin mRNA translation via an RNaseH-independent antisense mechanism (Boiziau, C; Kurfurst, R.; Cazanave, C; Roig, V.; Thuong, N.T. *Nucleic Acids Res.* 1991, *19,* 1113). Other examples shown also below are xylo-DNA, 2'-O-Me RNA and 2'-F RNA (reviewed in Sanghvi, Y.S.; Cook, P.D. in "Carbohydrate Modifications in Antisense Research", Sanghvi, Y.S.; Cook, P.D. (eds), *ACS Symposium Series,* vol. 580, pp. 1, American Chemical Society, Washington DC, 1994).

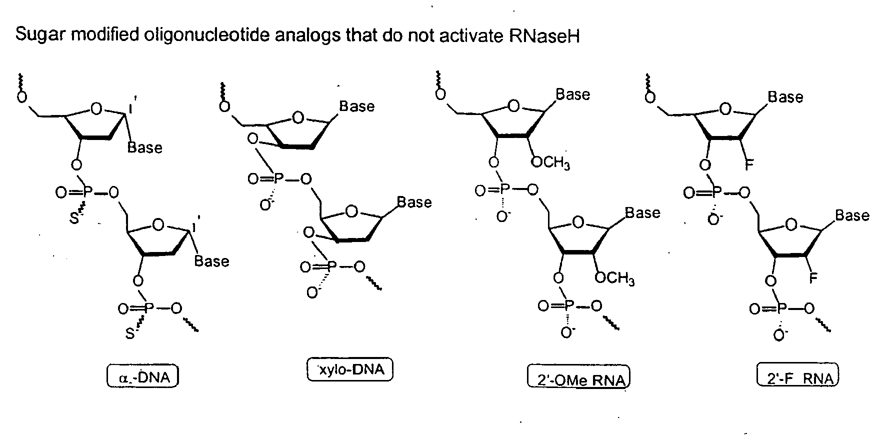

These analogues form stable duplexes with RNA targets, however, these duplexes are not substrates for RNaseH. To overcome this limitation, mixed-backbone oligonucleotides ("MBO") composed of either phosphodiester (PO) and phosphorothioate (PS) oligodeoxynucleotide segments flanked on both sides by sugar-modified oligonucleotide segments have been synthesized (Zhao, G. *et al., Biochem. Pharmacol. 1996,* 51, 173; Crooke, S.T. *et al. J. Pharmcol. Exp. Ther.* 1996, *277,* 923). Among the MBOs most studied to date is the [2'-OMe RNA]-[*PS DNA*] - [2'OMe RNA] chimera. The PS segment in the middle of the chain serves as the RNaseH activation domain, whereas the flanking 2'-OMe RNA regions increase affinity of the MBO strand for the target RNA. MBOs have increased stability *in vivo,* and appear to be more effective than phosphorothioate analogues in their biological activity both *in vitro* and *in vivo*. Examples of this approach incorporating 2'-OMe and other alkoxy substituents in the flanking regions of an oligonucleotide have been demonstrated by Monia *et al*. by enhanced antitumor activity *in vivo* (Monia, P.B.; Johnston, J.F.; Geiger, T.; Muller, M.; Fabbro, D. *Nature Med.* 1996, *2*, 668). Several pre-clinical trials with these analogues are ongoing (Akhtar, S.; Agrawal, S. "In vivo studies with antisense oligonucleotides" *TiPS* 1997, *18,* 12).

The synthesis of oligonucleotides containing hexopyranoses instead of pentofuranose sugars has also been reported (Herdewijn, P. *et al.,* in *"Carbohydrate Modifications in Antisense Research",* Sanghvi, Y.S.; Cook, P.D. (eds), *ACS Symposium Series,* vol. 580, pp. 80, American Chemical Society, Washington DC, 1994). A few of these analogues have increased enzymatic stability but generally suffer from a reduced duplex forming capability with the target sequence. A notable exception are 6'→4' linked oligomers constructed from 1,5-anhydrohexitol units which, due to their highly pre-organized sugar structure, form very stable complexes with RNA (van Aeroschot, A.C. *et al., Nucleosides* & *Nucleotides* 1997, *16*, 973). However, none of these hexopyranose oligonucleotide analogues have been shown to elicit RNaseH activity. Recently, oligonucleotides containing completely altered backbones have been synthesized. Notable examples are the peptide nucleic acids ("PNA") with an acyclic backbone (Nielsen, P.E. *in "Perspectives in Drug Discovery and Design",* vol. 4, pp. 76, Trainor, G.L. (ed.), ESCOM, Leiden, 1996). These compounds have exceptional hybridization properties, and stability towards nucleases and proteases. However, efforts to use PNA oligomers as antisense constructs have been hampered by poor water solubility, self-aggregation properties, poor cellular uptake, and inability to activate RNaseH. Very recently, PNA-[PS-DNA]-PNA chimeras have been designed to maintain RNaseH mediated cleavage via the PS-DNA portion of the chimera (Bergman, F; Bannworth, W.; Tam, S. *Tetrahedron Lett.* 1995, *36*, 6823; Van der Laan, A.C. *et al. Trav. Chim Pays-Bas* 1995, *114*, 295).

### Arabinonucleosides and Arabinonucleic Acids (ANA)

Arabinonucleosides are stereoisomers of ribonucleosides, differing only in the configuration at the 2'-position of the sugar ring. They have had a substantial impact on chemotherapy and as such they have been extensively used as antiviral and anticancer drugs (for a review, see: Wright, G.E.; Brown, N.C. *Pharmacol. Ther.* 1990, *47,* 447). β-D-Arabinofuranosylcytosine (ara-C) is the most successful nucleoside antileukemic agent and is widely used in combination therapy or at high doses as a single agent to treat patients with acute lymphoblastic and myeloblastic leukemias (Kufe, D.W.; Spriggs, D.R. *Semin. Oncol.* 1985, *12*, 34; Lauer *et al. Cancer* 1987, *60,* 2366).

Oligonucleotides constructed from arabinonucleotides ("arabinonucleic acids" or ANA, have been under investigation from various different aspects. ANA oligomers have been synthesized as pro-drugs in an attempt to improve the solubility of arabinonucleoside therapeutics. Incorporation of ara-C into DNA strands has also been the focus of research to understand the mechanism of action of this anticancer drug (Mikita, T.; Beardsley, G.P. *Biochemistry* 1988, **27,** 4698; Mikita, T.; Beardsley, G.P. *Biochemistry* 1994, **33,** 9195).

DNA strands containing arabinonucleosides have also been a subject of a number of structural studies. In the crystal, DNA duplexes containing araC adopt a normal B-type double helix with only small conformational perturbations at the araC-dG base pair (Chwang, A.K.; Sundaralingam, M. Nature 1973, **243,** 78; Teng, M. *et al. Biochemistry* 1989, *28,* 4923; Gao, Y.-G. *et al., Biochemistry* 1991, *30,* 9922). Mikita and Beardsley prepared DNA/DNA and DNA/RNA duplexes containing a single araC-G base pair to investigate the structural distortions caused by arabinonucleotides. They found that both the DNA duplex and the DNA/RNA hybrid can accommodate araC-dG(rG) base pair with only a moderate and equivalent loss of stability (Mikita, T.; Beardsley, G.P. *Biochemistry* 1994, *33,* 9195). Pfleiderer and co-workers synthesized an all-arabinose oligonucleotide mimicking a transfer RNA molecule (Resmini, M; Pfleiderer, W. *Helv. Chim. Acta* 1993, *76,* 158).

The association properties of uniformly modified oligoarabinonucleotides (ANA) were investigated by Giannaris and Damha and independently by Watanabe and co-workers (Giannaris, P.A.; Damha, M.J. *Can. J. Chem.* 1994, *72,* 909; Kois, P.; Watanabe, K.A. *Nucleic Acids Symposium Series* 1993, *29*, 215; Kois, P. *et al*. *Nucleosides* & *Nucleotides* 1993, *12*, 1093). Giannaris and Damha showed that oligomers of either purine or pyrimidine β-arabinonucleosides generally associate with complementary DNA and RNA with thermal stabilities comparable with those of the corresponding DNA strands (Giannaris, P.A.; Damha, M.J. *Can. J. Chem.* 1994, *72,* 909). For example, they showed that (a) an octaarabinoadenylate, ara-A₈ associated with poly ribo-U and poly deoxy-T; the melting temperature of the resulting complex was slightly higher than the corresponding complexes formed by the normal ribo-A₈ and deoxy-A₈ strands; (b) ara-C₈ and ara(UCU UCC CUC UCC C) associated with their complementary RNA strand, *albeit* with lower affinity relative to the corresponding unmodified strands; (c) ara-U₈ did not bind with poly rA under conditions where ribo-U₈ and deoxy-U₈ formed a complex with poly rA. Giannaris and Damha also reported that replacement of the normal phosphodiester (PO) linkage in ANA oligomers with phosphorothioate (PS) linkages had a severe destabilizing effect; the destabilization was greater than that observed when the PO linkages of a normal DNA strand were replaced with PS internucleotide linkages (Giannaris, P.A.; Damha, M.J. *Can. J. Chem.* 1994, *72*, 909). ANA oligomers displayed some stability against cleavage by snake-venom phosphodiesterase; however, they were rapidly degraded by nuclease P1, ribonuclease S1 and spleen-phosphodiesterase (Giannaris, P.A.; Damha, M.J. *Can. J. Chem.* 1994, *72*, 909).

Watanabe and co-workers incorporated 2'-deoxy-2'-fluoro-β-D-arabinofuranosylpyrimidine nucleosides (2'F-ara-N, where N=C, U and T) at multiple positions within a normal DNA chain and evaluated the hybridization properties of such (2'-F)ANA-DNA "chimeras" towards complementary DNA (Kois, P. *et al*. *Nucleosides & Nucleotides* 1993, *12*, 1093). They found that substitutions with 2'F-araU and 2'F-araC had a destabilizing effect on duplex stability, whereas substitution with 2'F-araT was stabilizing compared to unmodified oligodeoxynucleotide strands. The authors also reported that 2'F-araT₁₁ and 2'F-araU₁₁ oligomers were able to bind to the complementary DNA with equal or slightly better affinity compared to the control dT₁₁ (DNA) oligomer. Marquez and co-workers recently evaluated the self-association of a DNA strand in which two internal thymidines were replaced by 2'F-araT's (Ikeda *et al. Nucleic Acids Res.* 1998, *26*, 2237). They confirmed the findings of Watanabe and co-workers that internal 2'F-araT residues stabilize significantly the DNA double helix. The association of these (2'-F)ANA-DNA "chimeras" with complementary RNA (the typical antisense target) was not reported.

Recently, Noronha and Damha tested oligonucleotides based on β-D-arabinose for their ability to recognize duplex DNA, duplex RNA and DNA/RNA hybrids (Noronha, A.; Damha, M.J. *Nucleic Acids Res.* 1998, *26,* 2665). A pyrimidine oligoarabinonucleotide was shown to form triple-helical complexes with duplex DNA and hybrid DNA(purine)/RNA(pyrimidine). However, this oligoarabinonucleotide was found to bind with an affinity that was lower relative to the natural pyrimidine oligodeoxynucleotide or oligoribonucleotide controls.

Oligomers constructed from α-arabinofuranosylthymine (α-ara-T) exhibited a large decrease in melting temperature towards complement DNA when compared to the control DNA (β-dT) strand (Adams, A.D.; Petrie, C.R.; Meyer Jr., R.B. *Nucleic Acids Res.* 1991, *19,* 3647). On the other hand, the duplexes formed between either α-ara-T₁₅ or dT₁₅ and complementary RNA (poly-rA) were of similar strength. More recently, Wengel and co-workers reported the synthesis and association properties of DNA oligomers containing one and two β-2'-OMe-araT inserts (Gotfredsen, C.H.; Spielmann, P.; Wengel, J.; Jacobsen, J.P. *Bioconjugate Chem.* 1996, *7*, 680). These oligomers showed moderately lowered thermal stabilities towards both single stranded DNA and RNA, compared to unmodified DNA controls. The same authors reported that oligomers constructed from α-2'-OMe-araT units exhibited increased affinity towards the riboadenylate (RNA) target compared to normal DNA controls; however, α-2'-OMe araT strands did not display any advantage relative to the known α-dT oligomers. The susceptibility of the above duplexes to RNase H-mediated cleavage was not investigated.

### Activation of RNase H by Antisense Oligonucleotides

As described above, an important mechanism of action of antisense oligonucleotides is the induction of cellular enzymes such as RNaseH to degrade the target RNA (Walder, R.T.; Walder, J.A. *Proc. Natl. Acad. Sci. USA* 1988, *85*, 5011; Chiang *et al. J. Biol Chem.* 1991, *266,* 18162; Monia *et al. J. Biol. Chem.* 1993, *268*, 14514; Giles, R.V.; Spiller, D.G.; Tidd, D.M. *Antisense Res. & Devel.* 1994, *5*, 23; Giles *et* al. *Nucleic Acids Res.* 1995, *23*, 954). RNase H selectively hydrolyzes the RNA strand of a DNA/RNA heteroduplex (Hausen, P. ; Stein, H. *Eur. J. Biochem.* 1970, *14*, 279) . RNase H1 from the bacterium *Escherichia coli* is the most readily available and the best characterized enzyme. Studies with eukaryotic cell extracts containing RNase H suggest that both prokaryotic and eukaryotic enzymes exhibit similar cleavage properties (Monia *et al*. *J. Biol. Chem.* 1993, *268*, 14514; Crooke *et al. Biochem J.* 1995, *312*, 599; Lima, W.F.; Crooke, S.T. *Biochemistry* 1997, *36*, 390). *Escherichia coli* RNase H is thought to bind in the minor groove of the DNA/RNA double helix and to cleave the RNA by both endonuclease and processive 3'-to-5' exonuclease activities (Nakamura, H. *et al. Proc. Natl. Acad. Sci. USA* 1991, *88*, 11535; Federoff, O.Y.; Salazar, M.; Reid, B.R. *J*. *Mol. Biol.* 1993, *233*, 509; Daniher, A.T. *et al. Bioorg. & Med. Chem.* 1997; *5*, 1037). The efficiency of RNase H degradation displays minimal sequence dependence and is quite sensitive to chemical changes in the antisense oligonucleotide. For example, RNaseH degrades RNA in PS-DNA/RNA hybrids (Gao *et al*. *Mol. Pharmacol.* 1991, *41*, 223), but not in hybrids containing methylphosphonate-DNA, α-DNA, or 2'-OMe RNA antisense strands (For a review, see: Sanghvi, Y.S.; Cook, P.D. (eds), *ACS Symposium Series,* vol. 580, pp. 1, American Chemical Society, Washington DC, 1994). Furthermore, while *E*. *coli* RNaseH binds to RNA/RNA duplexes, it cannot cleave them, despite the fact that the global helical conformation of RNA/RNA duplexes is similar to that of DNA/RNA substrate duplexes ("A"-form helices)(Oda *et al. Nucleic Acids Res.* 1993, *21,* 4690). These results suggest that local structural differences between DNA/RNA (substrate) and RNA/RNA duplexes is responsible, at least in part, for substrate discrimination (Oda *et al. Nucleic Acids Res.* 1993, *21,* 4690; Lima, W.F.; Crooke, S.T. *Biochemistry* 1997, *36,* 390). In this regard it is interesting to note that HIV-1 reverse transcriptase (RT)-associated RNaseH cleaves both DNA/RNA and RNA/RNA duplexes; however, cleavage of the latter is at least 30-fold slower and occurs only when RT is artificially arrested (Gotte *et al., EMBO J.* 1995, *14*, 838).

### Arabinonucleic Acids as Activators of RNaseH Activity

An essential requirement in the antisense approach is that an oligonucleotide or its analogue recognize and bind tightly to its complementary target RNA. The ability of the resulting antisense oligomer/RNA hybrid to serve as a substrate of RNaseH is likely to have therapeutic value by enhancing the antisense effect relative to oligomers that are unable to activate this enzyme. Apart from PS-DNA (phosphorothioates), PS₂-DNA (phosphorodithioates), boranophosphonate-linked DNA, and MBO oligos containing an internal PS-DNA segment, there are no other examples of fully modified oligonucleotides that elicit RNaseH activity. For this reason, and because of the problems encountered with PS-oligonucleotides (e.g., non-antisense effects and potential risk of toxicity), we have designed alternative oligonucleotide analogues that selectively block gene expression through the activation of RNaseH activity. As a starting point, we felt that such analogues should (a) retain the natural β-D-furanose configuration, (b) possess the unmodified phosphate groups for solubility purposes, and (c) be able to mimic the conformation of DNA strands (e.g., with sugars puckered in the C2'-endo conformation). The latter requirement stems from the fact that the antisense strand of natural substrates is DNA, and as indicated above, its primary structure (and/or conformation) appears to be essential for RNaseH/substrate cleavage. Since the DNA sugars of DNA/RNA hybrids adopt primarily the C2'-endo conformation (Salazar, M.; Champoux, J.J.; Reid, B.R. *Biochemistry* 1993, *32,* 739; Salazar, M.; Federoff, O.Y.; Reid, B.R. *Biochemistry* 1996, *35*, 8126), we were interested in an oligonucleotide analog that favored this conformation. Analogues mimicking the RNA structure (*i.e.,* those that adopt the C3'-endo rather than the C2'-endo conformation) would not be suitable for evoking RNase H activity since it is known that RNA/RNA duplexes are generally not substrates of RNaseH. This prompted us to consider oligomers constructed from arabinonucleotides (i.e., the arabinonucleic acids or ANA). ANA is an stereoisomer of RNA differing only in the stereochemistry at the 2'-position of the sugar ring. ANA/RNA duplexes adopt a helical structure that is very similar to that of DNA/RNA substrates ("A"-form), as shown by similar circular dichroism spectra of these complexes. In addition, X-ray crystallographic studies on ara-C nucleosides and on DNA duplexes containing ara-C indicated that the arabinose sugar adopts the C1'-exo or the C2'-endo conformation; the latter conformation is found in the DNA sugars of DNA/RNA substrates. Furthermore, examination of molecular models of an A-type ANA/RNA duplex suggested that the β-2'-OH group of the arabinose strand is positioned within the major groove of the hybrid and thus should not interfere with RNase H's binding and catalytic processes. We also considered replacing the β-2'-OH by other electronegative substituents, *e.g.,* β-2'-fluorine, since strong stereoelectronic effects are expected to stabilize the C2'-endo form (Saenger, W. *Principles of Nucleic Acids Structure,* Cantor, C.R. (ed.), Springer-Verlag, N.Y., 1984; Marquez, V.E.; Lim, B.B.; Barchi, J.J., Jr.; Nicklaus, M.C., "Conformational studies of anti-HIV activity of mono- and difluorodideoxynucleosides", in *Nucleosides and Nucleotides as Antitumor and Antiviral Agents,* Chu, C.K.; Baker, D.C. (eds.), pp. 265-284, Plenum Press, N.Y., 1993). The possibility of an ANA oligomer activating RNaseH has not been reported.

It would be highly desirable to be provided with ANA oligomers and their analogues for sequence specific inhibition of gene expression via association to (and RNaseH mediated cleavage of) complementary messenger RNA.

### SUMMARY OF THE INVENTION

It is the purpose of this invention to provide ANA oligomers and their analogues for sequence specific inhibition of gene expression via association to (and RNaseH mediated cleavage of) complementary messenger RNA.

The present invention provides sugar-modified oligonucleotides that form a duplex with its target RNA sequence. The resulting duplex is a substrate for RNaseH, an enzyme that recognizes this duplex and degrades the RNA target portion. RNaseH mediated cleavage of RNA targets is considered to be a major mechanism of action of antisense oligonucleotides. The sugar-modified oligomers are defined in the claims.

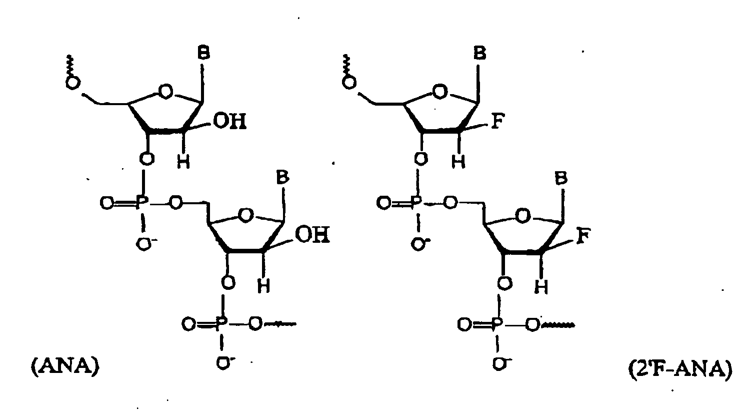

Prior to this invention, only the natural DNA (deoxyribonucleic acid phosphodiester) or deoxyribonucleic acid oligonucleotides based on phosphorothioate (PS-DNA), dithioate, and boranophosphonate backbones, had been reported to elicit RNaseH degradation of the target RNA. The present invention relates to the discovery that certain uniformly sugar-modified oligonucleotides, namely those based on 2'-deoxy-2'-fluoro-β-D-arabinonucleotides (i.e., 2'F-ANA oligomers), can activate RNaseH activity when duplexed to the target RNA sequences.

Also provided are oligonucleotides based on 2'-deoxy-2'-fluoro-β-D-arabinonucleosides (i.e., 2'F-ANA oligomers) that bind to duplex DNA with higher affinity relative to unmodified oligodeoxynucleotides.

Defined sequence oligoarabinonucleotides (ANA and 2'F-ANA) were prepared and found to inhibit the expression of a specific target mRNA that codes for the expression of a specific protein (luciferase). This inhibition was noted both in experiments that assessed inhibition of target protein expression in *in vitro* transcription/translation of the target (in the presence of a large excess of non-specific exogenous protein contributed by the *in vitro* transcription/translation system) and in experiments assessing target protein expression in intact cells.

In summary, our experiments establish that ANA oligomers serve as excellent models of antisense agents that have enhanced resistance to the action of degradative nucleases, bind to RNA through duplex formation, elicit RNase H activity, and inhibit *in vitro* and intracellular specific gene expression. Accordingly, ANA and its analogues have potential utility as therapeutics agents and/or tools for the study and control of specific gene expression in cells and organisms.

### BRIEF DESCRIPTION OF THE DRAWINGS

Figs. 1A-1B illustrate thermal melting curves of 18-bp heteroduplexes;
Figs. 2A-2C illustrate circular dichroic (CD) spectra of duplexes;
Fig. 3 illustrates thermal melting curves of triple helical complexes formed by the association of oligoarabinonucleotide SEQ ID NO:13 with DNA/DNA ("DD") and DNA/RNA ("DR") hairpin duplexes;
Fig. 4 illustrates gel mobility shift triplex assay under non-denaturing conditions;
Fig. 5 illustrates oligonucleotides with β-D-arabinose as sugar component elicit RNaseH degradation of complementary target RNA;
Fig. 6 illustrates homopolymeric oligonucleotides with 2'-F-β-D-arabinose as sugar component elicit RNaseH degradation of complementary target RNA;
Fig. 7 illustrates heteropolymeric oligonucleotides with 2'-F-β-D-arabinose as sugar component elicit RNaseH degradation of complementary target RNA;
Fig. 8 illustrates stability of oligonucleotides with 2'-F-β-D-arabinose as sugar component to degradation by serum nucleases;
Fig. 9 illustrates stability of oligonucleotides with 2'-F-β-D-arabinoee as sugar component to degradation by snake venom phosphodiesterase I;
Fig. 10 illustrates oligonucleotides based on β-D-arabinose and 2'-deoxy-2'-fluoro-β-D-arabinose show low nonspecific binding to cellular proteins;
Fig. 11 illustrates oligonucleotide inhibition of specific gene expression in an in vitro protein translation system; and
Fig. 12 illustrates oligonucleotide inhibition of luciferase gene expression in Hela X1/5 cells.

### DETAILED DESCRIPTION OF THE INVENTION

The present invention relates to oligonucleotides as defined in the claims and the therapeutic use of such compounds. It is the object of the present invention to provide a new oligonucleotide analogue that hybridizes to complementary nucleic acids which may be mRNA, viral RNA (including retroviral RNA). More particularly this invention relates to the use of oligonucleotides as defined in the claims to cleave complementary RNA via RNaseH activation. Other applications of this invention relates to the use of antisense oligonucleotides based on arabinonucleotides in combination with RNaseH as laboratory reagents for the sequence specific cleavage and mapping of RNA.

The oligonucleotides may be represented by the following Formula (I): 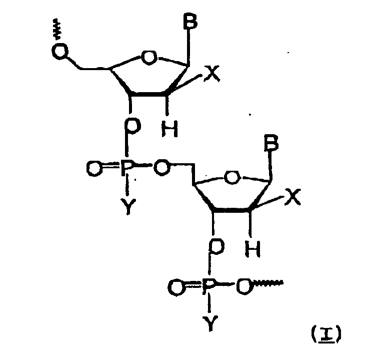 where B includes but it is not necessarily limited to a common purine or pyrimidine base such as adenine, guanine, cytosine, thymine, and uracil. The sugar is β-D-arabinofuranose, its mirror image enantiomer β-L-arabinofuranose, and the corresponding carbocyclic sugars (i.e., in which the ring oxygen at position 4' is replaced by a methylene or CH₂ group). The substituent at the 2' position of the sugar ring is Y at the internucleotide phosphate linkage is selected from the group consisting of hydroxyl, thiol, methyl, amino, alkylamino, dialkylamino, methoxy, and ethoxy. The ANA oligomers may also include modified sugars in part of the oligomer. The oligonucleotides may also include the 2'-deoxy-2',2"-difluoro-β-ribofuranose sugar (D or L configuration) in part or all of the oligomer (this structure is obtained by replacing the 2'-H atom in formula I with a fluorine atom, thus providing an oligonucleotide containing two fluorine atoms at carbon 2'). The oligonucleotides may also include stretches of ssDNA flanked by ANA segments (e.g., ANA-DNA-ANA, 2'F-ANA-DNA-2'F-ANA chimeras), or combination of ANA and 2'F-ANA segments (e.g., ANA-2'F-ANA chimeras). The ANA oligomers contain a sequence that is complementary to a specific sequence of a mRNA, or genomic viral RNA, such that the oligonucleotide can specifically inhibit protein biosynthesis, or virus replication (reverse transcription), respectively: A complementary target may also be duplex or single stranded DNA, such that the arabinonucleotide strand can specifically inhibit DNA replication and/or transcription. Partial modifications to the oligonucleotide directed to the 5' and/or 3'-terminus, or the phosphate backbone or sugar residues to enhance their antisense properties (e.g. nuclease resistance) are within the scope of the invention.

A group of oligonucleotides useful in this invention, are those wherein B is a natural base (adenine, guanine, cytosine, thymine, uracil); the sugar moiety is β-D-arabinofuranose; X is fluorine; Y is oxygen since these modifications give rise to oligomers that exhibit high affinity for single stranded RNA, single stranded DNA, and duplex DNA. In addition, these oligomers have been shown to meet the requirements necessary for antisense therapeutics. For example, they activate RNaseH activity, show resistance to cellular and serum nucleases, inhibit the expression of a specific target mRNA that codes for the expression of a specific protein in intact cells; and exhibit little (if any) nonspecific binding to cellular proteins; as such they may be potentially more effective *in vivo.*

The free β-D-arabinose pyrimidine (araU, araC) nucleoside monomers may be prepared from the corresponding ribonucleosides in good yields, and can be further elaborated to the corresponding 5'-O-monomethoxytrityl-2'-O-acetyl-3'-O-(β-cyanoethylphosphoramidite) derivatives suitable for solid-phase oligonucleotide synthesis (Giannaris, P.A.; Damha, M.J. *Can*. *J. Chem.* 1994, *72*, 909). The corresponding araA nucleoside is commercially available, and can be prepared readily from riboadenosine (via oxidation of the 2'-OH group and reduction of the 2'-keto group with a hydride source, e.g., Robins, M.J. *et al.* in *"Nucleosides, Nucleotides and their Biological Applications",* Rideaut, J.L.; Henry, D.W.; Beacham III, L.M. (eds.), pp. 279, Academic Press, Inc., 1993). The corresponding ara-G monomer can be prepared by the method of Pfleiderer and co-workers (Resmini, M.; Pfleiderer, W. *Helv. Chim. Acta* 1994, *77*, 429). The 3'-O-(β-cyanoethyl-N,N-diisopropylphosphoramidite) derivatives of 5'-MMT-2'-deoxy-2'-fluoro-β-D-arabinonucleosides (2'F-ara-C, 2'F-ara-A, 2'F-ara-G and 2'F-ara-T) may be synthesized following published procedures (Tann, C.H.; Brodfuehrer, P.R.; Brundidge, S.P.; Sapino, C. Jr.; Howell, H.G. *J*. *Org. Chem.* 1985, *50*, 3644; Howell; H.G.; Brodfuehrer, P.R.; Brundidge, S.P.; Benigni, D.A.; Sapino, C., Jr. *J*. *Org. Chem.* 1988, *53*, 85; Kois, P.; Tocik, Z.; Spassova, M.; Ren, W.-Y.; Rosenberg, I.; Farras Soler, J.; Watanabe, K.A. *Nucleosides & Nucleotides* 1993, *12,* 1093; Chou, T.-C.; Burchenal, J.H.; Fox, J.J.; Watanabe, K.A. *Chem. Pharm. Bull*. 1989, *37*, 336).

The protected arabinonucleoside monomers can be attached to the solid support by known methods. In a preferred embodiment, the solid support is long-chain alkylamine controlled pore glass, and the procedure of Damha *et al.* is used for its derivatization (Damha *et al. Nucleic Acids Res.* 1990, *18,* 3813).

The oligomers of this invention (constructed from 2-deoxy-2-fluoro-β-D-arabinose) exhibit a number of desirable properties:
(1) They were found to bind to and cleave single stranded RNA by activating RNaseH. Circular dichroism studies in solution showed that DNA/RNA hybrids (the natural substrate of RNase H) and ANA/RNA duplexes adopt a very similar helical structure that falls within the "A"-conformational family. The ability of RNaseH to degrade RNA in the ANA:RNA duplexes may be due, at least in part, to (a) the similarity of the structure of ANA/RNA to that of DNA/RNA duplexes, and (b) the fact that the 2'-substituent of the sugar ring is located in the major groove, where it does not interfere in RNase H's binding and catalytic processes. The 2'-fluorinated ANA derivatives in particular were found to have excellent affinity towards RNA targets, compared to normal DNA and phosphorothioate oligodeoxynucleotide strands.
(2) An oligonucleotides based on β-D-arabinose and containing four nucleobases (U, C, A and G) was found to hybridize to complementary RNA but not complementary single stranded DNA. This property suggests that these oligomers may be useful for targeting retroviral genomic RNA to inhibit early stages of virus replication, including reverse transcription. This high level of RNA specificity has previously been reported for other types of oligonucleotide analogs (e.g., 2',5'-linked RNA and 2',5'-linked DNA; Giannaris, P.A.; Damha, M.J., *Nucleic Acids Res.* 1993, 20, 4742; Alul, R.; Hoke, G.D. *Antisense Res. Dev.* 1995, *5*, 3), however, none of these oligonucleotides elicit RNaseH activity.
(3) Pyrimidine oligonucleotides constructed from 2'-deoxy-2'-fluoro-β-D-arabinonucleoside units were also found to hybridize to duplex DNA and DNA/RNA hybrids via triplex helix formation. The thermal stability of these triplexes are significantly higher than those formed by normal oligodeoxynucleotides. These results were unexpected given that the β-D-arabinose series produces triplexes with only modest stability (Noronha, A. ; Damha, M.J. *Nucleic Acids Res.* 1998, *26,* 2665).
(4) Results from metabolic stability studies indicate that the arabinose modification, particularly the β-D-arabinose (2'-OH) derivatives, confers greater resistance to degradation by both serum and cellular nucleases compared with natural strands (PO-DNA), although less than to phosphorothioate (PS-DNA) derivatives. Partial modifications to the ANA or 2'F-ANA oligonucleotide directed to the 5' and/or 3'-terminus, or the phosphate backbone or sugar residues to further enhance nuclease resistance are within the scope of the invention.
(5) ANA and 2'F-ANA show little (if any) non-specific binding to cellular proteins and serum proteins. This property results in a significantly improved interaction of arabinooligonucleotides with target RNA in the presence of cell proteins compared to the phosphorothioate analogs.

These properties combined establish that ANA and 2'F-ANA oligomers serve as excellent models of antisense agents that have resistance to the action of degradative nucleases, bind to RNA and single stranded DNA through duplex formation, bind to duplex DNA through triplex formation, and elicit RNase H activity. Consequently, antisense oligonucleotide constructs containing arabinose and their analogues should serve as therapeutics and/or valuable tools for studying and controlling gene expression in cells and organisms.

The following examples are given by way of illustration of the present invention. The examples are not intended in any way to limit the scope of the invention.

### EXAMPLE 1

### Preparation of Oligonucleotides containing β-D-Arabinofuranoses

Oligoarabinonucleotides (Formula I; X= OH, Y= O⁻) were synthesized using standard phosphoramidite chemistry and 3'-ara-C(Bz)-long-chain alkylamine controlled pore glass solid support (lcaa-CPG; 500 Å; 1 µmol scale). The required monomers, namely 5'-MMT-2'-OAc-3'-O-(β-cyanoethyl-N,N-diisopropylphosphoramidite) derivatives of ara-A(Bz), ara-C(Bz) and ara-U were synthesized by the method of Damha *et al.* (Damha, M.J.; Usman, N.; Ogilvie, K.K., *Can. J. Chem.* 1988, *67*, 831; Giannaris, P.A.; Damha, M.J.; *Can. J. Chem.* 1994, *72*, 909). The corresponding ara-G (N²-*i*-Bu, O⁶-NPE) monomer was prepared by a modification of the procedure of Resmini *et al.* (Resmini, M.; Pfleiderer, W. *Helv. Chim. Acta* 1994, *77*, 429). Thus, the monomers were dissolved to 0.12 M in anhydrous acetonitrile. Prior to chain assembly, the support (1 µmol) was treated with the capping reagents, acetic anhydride/N-methylimidazole/4-dimethylaminopyridine (Damha, M.J.; Ogilvie, K.K. in *Methods in Molecular Biology, 20, Protocols for Oligonucleotides and Analogs: Synthesis and Properties,* Agrawal, S. (ed.), pp. 81, The Humana Press, Inc. Totawa, N.J., 1993). Chain assembly of sequences was carried out using an Applied Biosystem DNA synthesizer (Model 381A) as follows: (i) detritylation: 3% trichloroacetic acid in dichloroethane delivered in 100 s (+ 40 s 'burst') steps. The eluate from this step was collected and- the absorbance at 478 nm (MMT+, arabino sequences) measured to determine the average coupling reaction yield (ca. 60-90%); (b) nucleoside phosphoramidite coupling time of 7.5 min; (c) capping: 1:1 (v/v) of acetic anhydride/collidine/THF 1:1:8 (solution A) and 1-methyl-1H-imidazole/THF 16:84 (solution B) delivered in 15 s + 35 s "wait" steps; (d) oxidation: 0.1M iodine in THF/water/pyridine 7:2:1, delivered in 20 s + 35 s "wait" step. The 5'-terminal trityl group was removed by the synthesizer and the oligomers were then removed from the support and deprotected by treatment of the CPG with a solution containing concentrated ammonium hydroxide/ethanol (3:1 v/v, 1 mL) for two days at room temperature. The ammonium hydroxide/ethanol solution was evaporated and the crude product purified by preparative polyacrylamide gel electrophoresis (PAGE) followed by gel filtration (desalting) on a Sephadex G-25 column. For sequences containing ara-G units, it was necessary to subject the partially protected oligomer to an additional step; that is, following the ammonia treatment and evaporation step, the oligomer was treated with a solution of 1M tetra-n-butylammonium fluoride (50 µL, r.t., 16 h) in THF. This step cleaves the p-nitrophenylethyl protecting group at the O6-position of guanine residues. This solution is then quenched with water (1 mL) and desalted via size exclusion chromatography (Sephadex G-25 column). Purification is then carried out by gel electrophoresis as described above, and its molecular weight confirmed by MALDI-TOF mass spectrometry. The yield, base sequence, hybridization properties of the oligomers synthesized are given in Table 1.

**TABLE 1**

| **Base composition, yield and properties of oligoarabinonucleotides (ANA)** | | | | |
|---|---|---|---|---|
| | | | **Melting Temperature (°C)**^{**c**} | |
| **Base sequence of Oligonucleotide**^{**a**} | **SEQ ID NO:** | **Yield**^{**b**} | **RNA target** | **DNA target** |
| ara(AGC UCC CAG GCU CAG AUC) | 1 | 5 | 44 | 26^{d} |
| ara(AAA AAA AAA AAA AAA AAA) | 2 | 10 | 26 | 45 |
| ara(UUU UUU UUU UUU UUU UUU) | 3 | 9 | n.o.^{e} | n.o.^{e} |
| ara(UUA UAU UUU UUC UUU CCC) | 4 | 10 | 32 | <15^{d} |
| ara(AUA UCC UUG UCG UAU CCC) | 5 | 8 | 47 | n.m.^{f} |

| | | | | |
|---|---|---|---|---|
| ^{a} Sequence is written in the 5' → 3' direction; ^{b} Optical density units (A₂₆₀ nm); ^{c} Buffer containing 140 mM KCl, 1 mM MgCl₂, 5 mM Na₂HPO₄ (pH 7.2); ^{d} weak and broad transition ^{e} no melt curve or sharp transition observed; ^{f} not measured. | | | | |

Presently only the 5'-DMT, 2'-OAc, ara-C (Bz) 3'-phosphoramidite derivative and 3'-ara-C (Bz) long-chain alkylamino controlled pore glass (lcaa-CPG) are commercially available. Of the free (unprotected) nucleosides, only ara-C, ara-U and ara-A are commercially available.

### EXAMPLE 2

### Preparation of Oligonucleotides containing 2-Deoxy-2-Fluoro-β-D-Arabinose sugars

Oligoarabinonucleotide synthesis (Formula I; X= F, Y = O⁻) was performed on an Applied Biosystem DNA synthesizer (model 381A) using the phosphoramidite approach. Oligomers were prepared on a 1.0 µmol scale using lcaa-CPG solid support bearing 3'-terminal 2'-deoxy-2'-fluoro-β-D-arabinonucleosides. Coupling yields ranged from 60 to 100% (average ca. 80%) as monitored by the release of the MMT cation. The required 3'-O-(β-cyanoethyl-N,N-diisopropylphosphoramidite) derivatives of 5'-MMT-2'-deoxy-2'-fluoro-β-D-arabinonucleosides (2'F-ara-C, 2'F-ara-A, 2'F-ara-G and 2'F-ara-T) were synthesized by published procedures (Tann, C.H.; Brodfuehrer, P.R.; Brundidge, S.P.; Sapino, C. Jr.; Howell, H.G. *J. Org. Chem.* 1985, *50*, 3644; Howell; H.G.; Brodfuehrer, P.R.; Brundidge, S.P.; Benigni, D.A.; Sapino, C., Jr. *J. Org. Chem.* 1988, *53*, 85; Kois, P.; Tocik, Z.; Spassova, M.; Ren, W.-Y.; Rosenberg, I.; Farras Soler, J.; Watanabe, K.A. *Nucleosides* & *Nucleotides* 1993, *12*, 1093; Chu, C.K.; Matulic-Adamic, J.; Huang, J.-T.; Chou, T.-C.; Burchenal, J.H.; Fox, J.J.; Watanable, K.A. *Chem. Pharm. Bull*. 1989, *37*, 336). Thus, the monomers were dissolved to 0.10 M in anhydrous acetonitrile. Prior to chain assembly, the support (1 µmol) was treated with the capping reagents, acetic anhydride/N-methylimidazole/4-dimethylamino pyridine (Damha, M.J.; Ogilvie, K.K. in *Methods in Molecular Biology, 20, Protocols for Oligonucleotides and Analogs*: *Synthesis and Properties,* Agrawal, S. (ed.), pp. 81, The Humana Press, Inc. Totawa, N.J., 1993). Chain assembly of sequences was carried out as follows: (i) detritylation: 3% trichloroacetic acid in dichloroethane delivered in 140 s (+ 80 s 'burst') steps. (b) nucleoside phosphoramidite coupling time of 10 min; (c) capping of 5'-hydroxyl groups: 1:1 (v/v) of acetic anhydride/collidine/THF 1:1:8 (solution A) and 1-methyl-1*H*-imidazole/THF 16:84 (solution B) delivered in 15 s + 35 s "wait" steps; (d) oxidation of phosphite triester linkage: 0.1M iodine in THF/water/pyridine 7:2:1, delivered in 20 s + 35 s "wait" step. The 5'-terminal trityl group was removed by the synthesizer and the oligomers were then removed from the support and deprotected by treatment of the CPG with a solution containing concentrated ammonium hydroxide/ethanol (3:1 v/v, 1 mL) for two days at room temperature. The ammonium hydroxide/ethanol solution was evaporated and the crude product purified by preparative polyacrylamide gel electrophoresis (PAGE) followed by gel filtration (desalting) on a Sephadex G-25 column. Molecular weight of oligomers were confirmed by MALDI-TOF mass spectrometry. The yield, base sequence, and hybridization of the oligomers synthesized are given in TABLE 2.

**TABLE 2**

| **Base composition, yield and properties of 2'-F-oligoarabinonucleotides (2'F-ANA)** | | | | |
|---|---|---|---|---|
| | | | **Melting Temperature (°C)**^{**c**} | |
| **Base sequence of Oligonucleotide**^{**a**} | **SEQ ID NO:** | **Yield**^{**b**} | **RNA target** | **DNA target** |
| 2'F-ara(AGC TCC CAG GCT CAG ATC) | 6 | 11 | 86 | 68 |
| 2'F-ara(TTT TTT TTT TTT TTT TTT) | 7 | 15 | 52 | 55 |
| 2'F-ara(AAA AAA AAA AAA AAA AAA) | 8 | 27 | 30 | 63 |
| 2'F-ara(TTATATTTTTTCTTTCCC) | 9 | 15 | 64 | 54 |
| 2'F-ara(ATA TCC TTG TCG TAT CCC) | 10 | 21 | 76 | n.m.^{d} |

| | | | | |
|---|---|---|---|---|
| ^{a} Sequence is written in the 5' → 3' direction; ^{b} Optical density units (A₂₆₀ nm); ^{c}Buffer containing 140 mM KCl, 1 mM MgCl₂, 5 mM Na₂HPO₄ (pH 7.2); ^{d} Not measured. | | | | |

### EXAMPLE 3

### Association Properties of Uniformly Modified Oligonucleotides possessing β-D-Arabinose and β-D-2-Fluoro-2-Deoxyarabinose Sugars Binding to single stranded DNA and RNA Targets

The ability of oligonucleotides to hybridize to single-stranded nucleic acids to give a double- helical complex is crucial for their use as antisense therapeutic agents. The formation of such a complex involves stacking and hydrogen bonding interactions between the base chromophores, a process which is accompanied by a reduction in UV absorption ("hypochromicity"). When the temperature of the solution containing the double-helical complex is gradually raised, the hydrogen bonds break and the duplex dissociates into single strands. This reduces the amount of base-base interactions and hence leads to a sudden increase of the UV absorbance. The temperature at which the double-helical complex dissociates, or more precisely, the point at which half the population exists as complex and the remaining half as single strands, is termed the "melting temperature" (*T*ₘ). Thus a common technique used in nucleic acid chemistry to investigate duplex formation (and its strength) involves mixing equimolar amounts of the strand of interest together, incubating at low temperature to allow strands to anneal, and then observing the UV-absorption at 260 nm (absorption maxima) as a function of temperature. The result is an absorbance versus temperature plot, or sigmoidal "melt profile" or "melting curve" from which the *T*ₘ (the midpoint of the raise of the melt curve) is calculated (Wickstrom, E.; Tinoco, I. Jr. *Biopolymers* 1974, *13*, 2367). Circular dichroism (CD) is another powerful optical technique for the study of nucleic acid structure and conformation. The CD spectrum usually includes a region of rapid change (Cotton effects) with respect to wavelength (200-350 nm region). The signs, absolute intensity and position of the Cotton effects are particularly sensitive to chemical composition and three-dimensional structure of the nucleic acid complexes. CD measurements can therefore be applied to determine global helical conformation (or helix type) as well as to investigate structural changes (e.g., helix-to-coil transitions) as a function of temperature (Bloomfield, V.A.; Crothers, D.; Tinoco, Jr., I. *"Physical Chemistry of Nucleic Acids",* Harper and Row, N.Y., 1974; Ts'o, P.O.P. (ed.), "Basic Principles in Nucleic Acid Chemistry", vol. 1 and 2, Academic Press, N.Y., 1974).

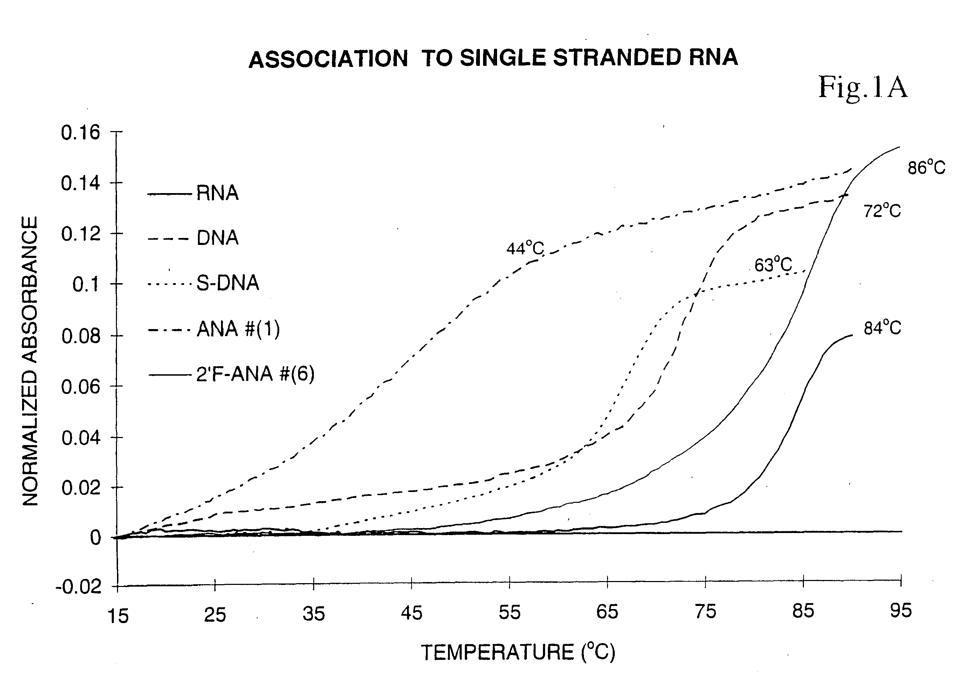

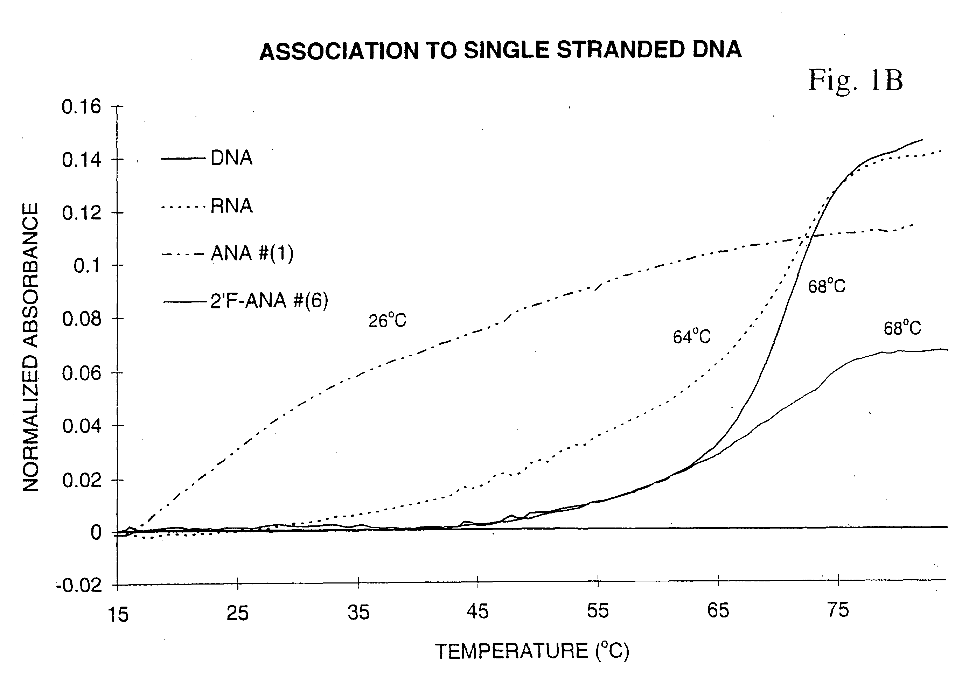

The binding properties of oligonucleotides (SEQ ID NO:1, and SEQ ID NO:6) (for base sequences see Tables 1 and 2) with complementary DNA and RNA single strands were evaluated in a buffer containing 140 mM KCl, 1 mM MgCl₂, 5 mM Na₂HPO₄ (pH 7.2), which is representative of intracellular conditions (Alberts, B. *Molecular Biology of the Cell,* pp. 304, Garland, N.Y., 1989). Molar extinction coefficients for oligoarabinonucleotide strands were calculated using the nearest-neighbor approximation, and were assumed to be the same as those of normal strands (Puglisi, J.D.; Tinoco, I. Jr. Methods in Enzymology, Dahlberg, J.E.; Abelson, J.N. (eds.), *180,* pp. 304, Academic Press, S.D., 1989). Thermal denaturation curves were acquired at 260 nm from 5°C to 90°C (rate of heating: 0.5°C/min), at a concentration of approximately 2.8 µM of each strand. Melting temperatures (*T*ₘ) were calculated from first-derivative plots of absorbance versus temperature. Thermal denaturation curves of the heteroduplexes are shown in Figs. 1A & 1B. Oligoarabinonucleotides ANA (SEQ ID NO:1), 2'F-ANA (SEQ ID NO:6), and control DNA, PS-DNA and RNA oligonucleotides were hybridized to (Fig. 1A) complementary single-stranded RNA, and (Fig. 1B) complementary single-stranded DNA.

The results (Fig. 1A) show that the arabinonucleotide of mixed base sequence, ANA (SEQ ID NO:1), has the ability to form a stable heteroduplex with its RNA complement, exhibiting a *T*ₘ of 44°C, compared to 72°C for the corresponding natural DNA/RNA heteroduplex. A 1:1 mixture of ANA (SEQ ID NO:1) and its DNA complement showed a much weaker and broader transition suggesting that, under the conditions used, SEQ ID NO:1 does not bind to single stranded DNA (Fig. 1B). The data shown in Fig. 1A and Table 2 also show that interaction of 2'F-oligoarabinonucleotides with complement RNA results in the formation of heteroduplexes that are of superior thermal stability relative to the complexes formed by the natural (PO-DNA) and thioate (PS-DNA) strands. For example, the *T*ₘ of 2'F-araA₁₈ (SEQ ID NO:8)/ rU₁₈ heteroduplex was 30.2°C, compared to 25.4°C for the natural dA₁₈/ rU₁₈ heteroduplex. Similarly, the *T*ₘ of the 2'F-araT₁₈(SEQ ID NO:7)/rA₁₈ heteroduplex was 43.9°C, which represents an increase in *T*ₘ of ca. 5°C relative to the natural dT₁₈/rA₁₈ heteroduplex (*T*ₘ 39°C). Also, the mixed base heteroduplex formed by the association of 2'F-ANA (SEQ ID NO:6) and its target RNA sequence is thermally more stable (*T*ₘ 86°C) than the corresponding of PO-DNA/RNA and PS-DNA/RNA duplexes (Fig. 1A). In contrast to the behavior observed for ANA sequence (SEQ ID.NO:1) (which exhibited selective binding to RNA), the 2'F-ANA oligonucleotides (e.g. SEQ ID NO:6) bind strongly to both single stranded complementary DNA and RNA. In fact, 2'F-araA₁₈ (SEQ ID NO:8) formed a more stable heteroduplex with single stranded complementary DNA (dT₁₈) than with RNA (rU₁₈), i.e., *T*ₘ 63.3°C and 30.2°C, respectively (Table 2). This amounts to a binding selectivity of Δ*T*ₘ = +33°C . The selective binding to single stranded PO-DNA (over RNA) was also observed for the natural dA₁₈ strand, although in this case the selectivity observed was less (Δ*T*ₘ = +20°C).

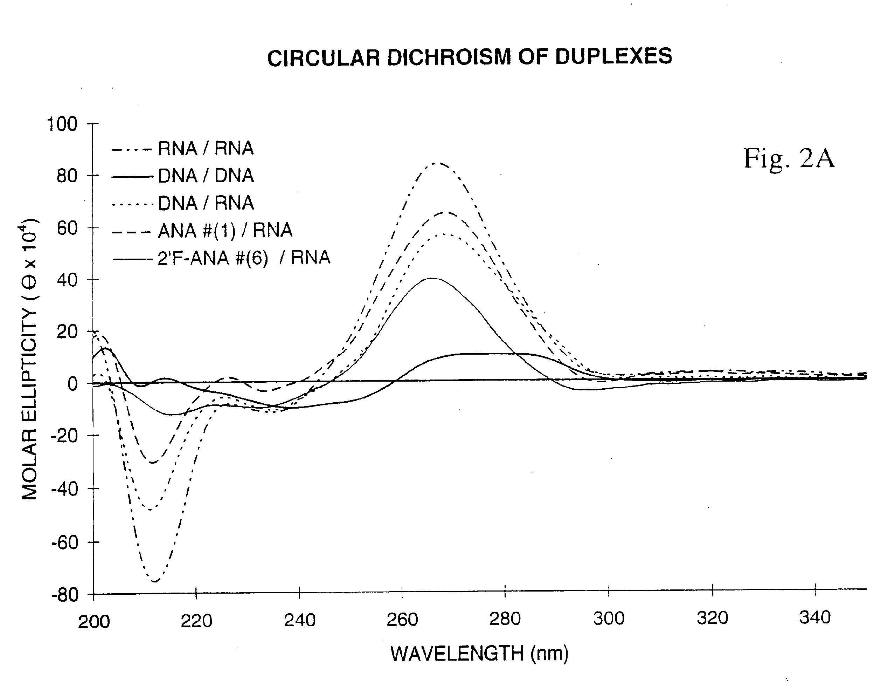

As shown in Fig. 2A, the CD spectra of the heteroduplexes ANA (SEQ ID NO:1)/RNA and 2'F-ANA (SEQ ID NO:6)/RNA, closely resembled those of the corresponding DNA/RNA control duplexes (the normal substrate of RNaseH), suggesting that all of these complexes share the same helical conformation. The spectral features observed are characteristic of a "A"-type helix, a structure that appears to be important in the recognition of DNA/RNA substrates by RNase H (Lima, W.F., Crooke, S.T. *Biochemistry* 1997, *36,* 390). The fact that ANA/RNA and 2'F-ANA/RNA heteroduplexes are substrates of RNaseH (see Examples 5 and 6) is fully consistent with this notion. The CD spectra of the DNA/DNA duplex (of the same base sequence) is very different, and is characteristic of the "B-form" helical conformation. The CD spectra of the A-form RNA/RNA control duplex is also shown.

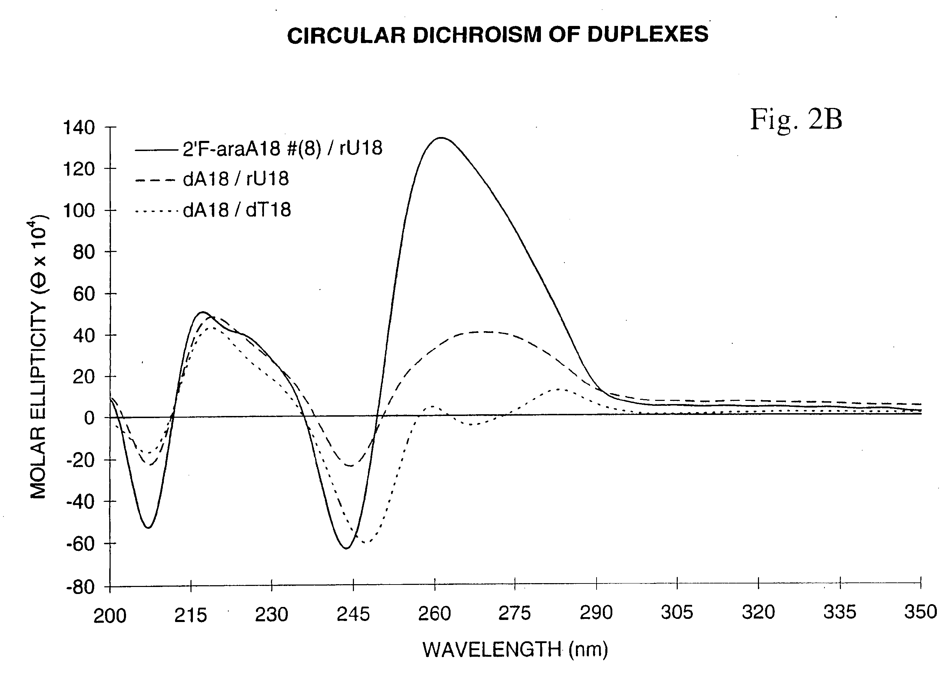

Fig. 2B shows the CD spectra of 2'F-araA₁₈ (SEQ ID NO:8)/rU₁₈ and dA₁₈/rU₁₈ duplexes, as well as the corresponding dA₁₈/ dT₁₈ duplex. The first two duplexes exhibit a similar CD profiles (i.e., peak pattern and peak position) that is characteristic of the A-helix conformation, whereas the spectrum of the DNA/DNA duplex (dA₁₈/ dT₁₈) falls into a pattern that is typical of "B-form" helices.

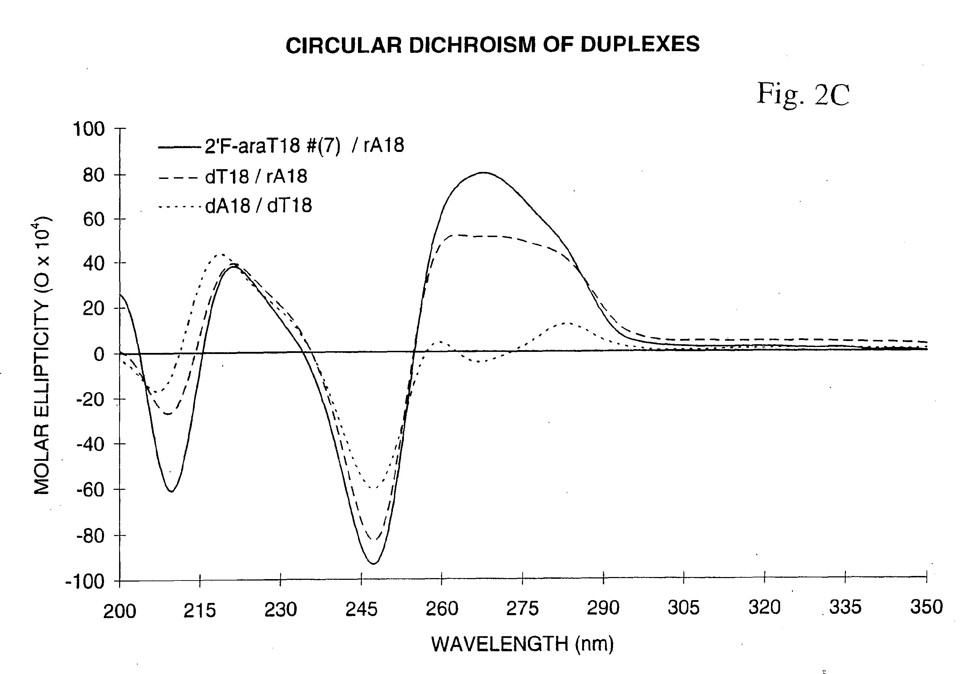

The same conclusions can be reached from the spectra shown in Fig. 2C. The CD spectra of the 2'F-araT₁₈ (SEQ ID NO:7)/ rA₁₈ duplex displays very similar spectral features of the normal dT₁₈/rA₁₈ hybrid, also typical of the A-form conformation. The spectra of the DNA/DNA duplex, dA₁₈/dT₁₈ (B-form), is also shown for comparison.

### EXAMPLE 4

### Association Properties of Oligonucleotides possessing 2-Deoxy-2-Fluoro-β-D-Arabinose Sugars Binding to DNA Duplexes and DNA/RNA Hybrids

To study the interaction between oligomers of 2'-deoxy-2'-fluoroarabinonucleotides and DNA/DNA and DNA/RNA duplexes, the experimental design of Roberts and Crothers was adopted (Roberts, R.W.; Crothers, D.M. *Science* 1992, *258*, 1463). The target duplexes are the following purine-pyrimidine hairpins:

| | | | |
|---|---|---|---|
| | 5'-GGAGAGGAGGGA | T | |
| DNA/DNA | | | T |
| (SEQ ID NO:11) | | | T |
| | 3'-CCTCTCCTCCCT | T | |
| | 5'-GGAGAGGAGGGA | T | |
| DNA/*RNA* | | | T |
| (SEQ ID NO:12) | | | T |
| | *3'-CCUCUCCUCCCU* | T | |

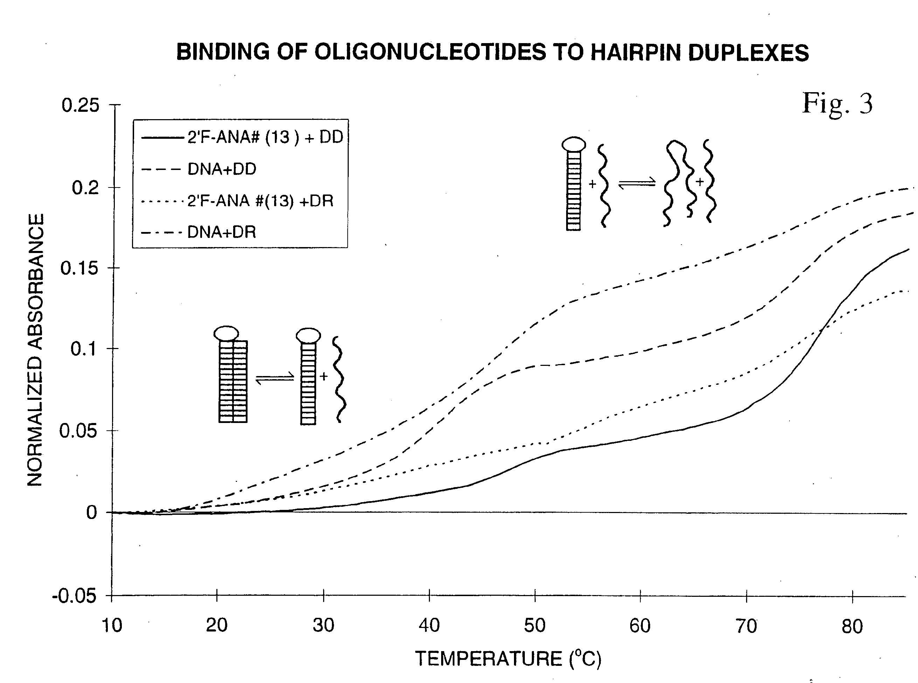

Triplex-helix formation can occur when an oligonucleotide binds in the major groove of the targeted duplexes (Le Doan, T. *et al*. *Nucleic Acids Res.* 1987, *238*, 645; Moser; H.E.; Dervan, P.B. *Science* 1987, *238*, 645). The oligopyrimidine strand containing 2'-deoxy-2'-fluoro-arabinose sugars, i.e., 2'F-ara(CCT CTC CTC CCT) (SEQ ID NO:13) was used to assess triple helix formation with the above hairpin duplexes. For the purpose of comparisons, the association of the corresponding oligodeoxyribopyrimidine ("DNA", 2'-deoxy-β-D-ribose) and oligoarabinopyrimidine ("ANA", β-D-arabinose) sequences were also examined. The ability of these oligomers to form triple helices was determined from ultraviolet spectroscopic melting experiments (as described in Example 3) and native gel electrophoresis, in a solution containing 100 mM sodium acetate and 1 mM ethylenediamine tetraacetate (EDTA), pH 5.5. Molar extinction coefficients for oligonucleotides were calculated from those of the mononucleotides and dinucleotides according to nearest-neighbouring approximations (Puglisi, J.D.; Tinoco, I. Jr. Methods in Enzymology, Dahlberg, J.E.; Abelson, J.N. (eds.), *180,* pp. 304, Academic Press, S.D., 1989). The values for the hybrid hairpin was assumed to be the sum of their DNA plus RNA components: DNA/DNA, 26.5; DNA/RNA, 27.1; (units= 10⁴ M⁻¹ cm⁻¹). The molar extinction coefficient for the 2'-fluoroarabinonucleotide strand SEQ ID NO:13 was assumed to be the same as a normal DNA strand (9.6 × 10⁴ M⁻¹ cm⁻¹ units). Complexes were prepared by mixing equimolar amounts of interacting strands, e.g., 2'F-ANA (SEQ ID NO:13) + hairpin DNA/DNA or DNA/RNA, and lyophilizing the resulting mixture to dryness. The resulting pellet was then re-dissolved in 100 mM NaOAc/ 1 mM EDTA buffer (pH 5.5). The final concentration was 2 µM in each strand. The solutions were then heated to 80°C for 15 min, cooled slowly to r.t., and stored at 4°C overnight before measurements. Prior to the thermal run, samples were degassed by placing them in a speed-vac concentrator (2 min). Denaturation curves were acquired at 260 nm at a rate of heating of 0.5°C/min. Melting temperatures (*T*ₘ) were calculated from the first derivative of the melting curves. The results of the melting experiments are shown in Fig. 3.

The results show that when 2'F-ANA (SEQ ID NO:13) was mixed with an equimolar concentration of hairpin DNA/DNA (SEQ ID NO:11) or DNA/RNA (SEQ ID NO:12), a biphasic transition was observed upon heating the solution from 10°C to 90°C. The low temperature transition is assigned to the dissociation of 2'F-ANA (SEQ ID NO:13) from the target hairpins (Roberts, R.W.; Crothers, D.M. *Science* 1992, *258*, 1463). The high temperature transition corresponds to the melting of the hairpin duplexes since it was also observed when a solution of hairpin duplex alone was heated under identical conditions. The data show that *T*ₘ values for low temperature transitions resulting from mixtures of SEQ ID NO:13 (2'-deoxy-2'-fluoro-β-D-arabinose oligomer) + hairpins are considerable higher than *T*ₘ values for transitions from the corresponding ANA (β-D-arabinose oligomer) + hairpin, or DNA (2'-deoxy-β-D-ribose oligomer) + hairpin mixtures. For example, as can be seen from the melting curves shown in Fig. 3, the *T*ₘ for the dissociation of 2'F-ANA strand SEQ ID NO:13 from the DNA/DNA hairpin (DD, SEQ ID NO:11) is 49°C, compared to 34°C and 40°C, for the dissociation of the ANA (β-D-arabinose; not shown) and DNA (2'-deoxy-β-D-ribose) oligonucleotides, respectively. Similarly, the first transition for the triplex formed by 2'F-ANA (SEQ ID NO:13) and DNA/RNA hairpin (SEQ ID NO:12) was 53°C, compared to 43°C and 45°C, for the corresponding triplexes formed by the ANA (β-D-arabinose; not shown) and DNA (2'-deoxy-β-D-ribose) strands, respectively.

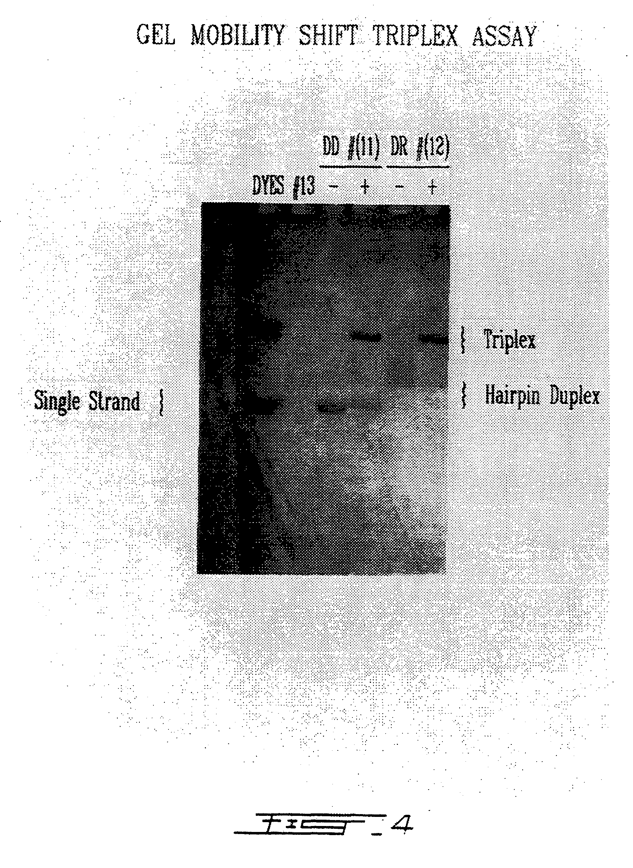

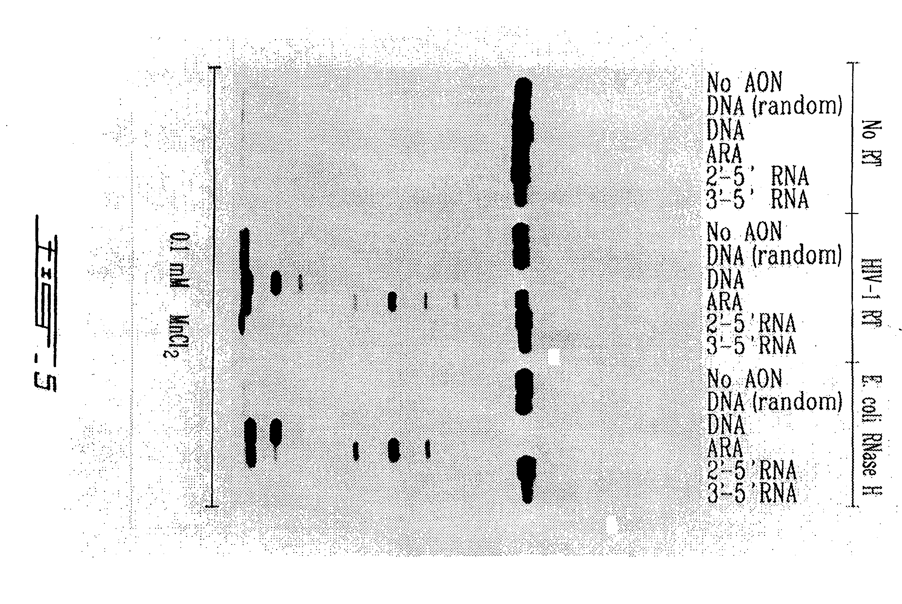

The equilibrium between single-, double-, and triple-stranded species of 2'F-ANA (SEQ ID NO:13) + hairpin mixtures was also directly monitored by polyacrylamide gel electrophoresis (Fig. 4). Fig. 4 shows a photograph of a polyacrylamide gel of single stranded oligo-2'F-arabinopyrimidine SEQ ID NO:13 and target hairpins DNA/DNA (SEQUENCE ID NO:11) ("DD") and DNA/RNA (SEQUENCE ID NO:12) ("DR"), and 1:1 ratios of SEQ ID NO:13:hairpins. The first lane shows marker dyes xylene cyanol (XC) and bromophenol blue (BPB). The next lane shows the SEQ ID NO:13 strand, whereas the "DD (-)" lane shows the DNA/DNA hairpin (SEQ ID NO:11). The SEQ ID NO:13:DD triplex is clearly seen in the "DD (+)" lane, which contains a 1:1 molar mixture of 2'F-ANA (SEQ ID NO:13) and "DD". The DNA/RNA hairpin (SEQ ID NO:12) is visible in the "DR (-)" lane. The triplex SEQ ID NO:13:DR triplex is clearly visible in the "DR (+)" lane, which consists of a 1:1 mixture of 2'F-ANA (SEQ ID NO:13) and "DR". Gels were visualized by UV-shadowing. Base sequence of single strand 2'F-ANA (SEQ ID NO:13) and hairpins DD (SEQ ID NO:11) and DR (SEQ ID NO:12) and experimental conditions are given above in Example 4.

This method provides a convenient way to monitor triplex formation and to qualitative check on the stoichiometry of interaction the strands (Kibler-Herzog, L. *et al. Nucleic Acids Res.* 1990, *18,* 3545). The results in Fig. 4 show that the 2'F-ANA strand (SEQ ID NO:13), hairpins, and triple-helical complexes can be separated with excellent resolution on a polyacrylamide gel at low temperature. As can be seen from the gel results, the triple-helical complex is nearly quantitatively formed at a 1:1 molar ratio of 2'F-ANA (SEQ ID NO:13):hairpin. This is in contrast to the incubation of ANA (D-arabinose strand) and hairpin (1:1 molar ratio) which, under the same conditions, gave a mixture of ANA + hairpin + triplex (see Noronha, A.; Damha, M.J. *Nucleic Acids Res.* 1998, *26*, 2665). This is in complete agreement with the *T*ₘ results which indicates that the SEQ ID NO:13 (2'-deoxy-2'-fluoro-β-D-arabinose) strand has a significantly higher affinity for the DNA/DNA and DNA/RNA hairpin duplexes relative to the ANA (β-D-arabinose) strand.

### EXAMPLE 5

### Induction of Ribonuclease H (RNaseH) Activity by Oligonucleotides possessing β-D-Arabinose as Sugar Component

Defined-sequence oligonucleotides, 18-units in length, were used in these experiments, i.e.,
5'-d(AGCTCCCAGGCTCAGATC) -3' "DNA" (SEQ ID NO:14)
5'-ara(AGCUCCCAGGCUCAGAUC)-3' "ARA" (SEQ ID NO:1)
5'-ribo(AGCUCCCAGGCUCAGAUC)-3' "3',5'-RNA" (SEQ ID NO:15)
5'-ribo(AGCUCCCAGGCUCAGAUC)-3' "2',5'-RNA" (SEQ ID NO:16)

These oligomers are complementary to a sequence within the R region of HIV-1 genomic RNA. The target RNA used was a synthetic 18 nt 3',5'-RNA oligonucleotide, identical to the sequence within the HIV R region, and exactly complementary to the sequence of the above oligonucleotides.

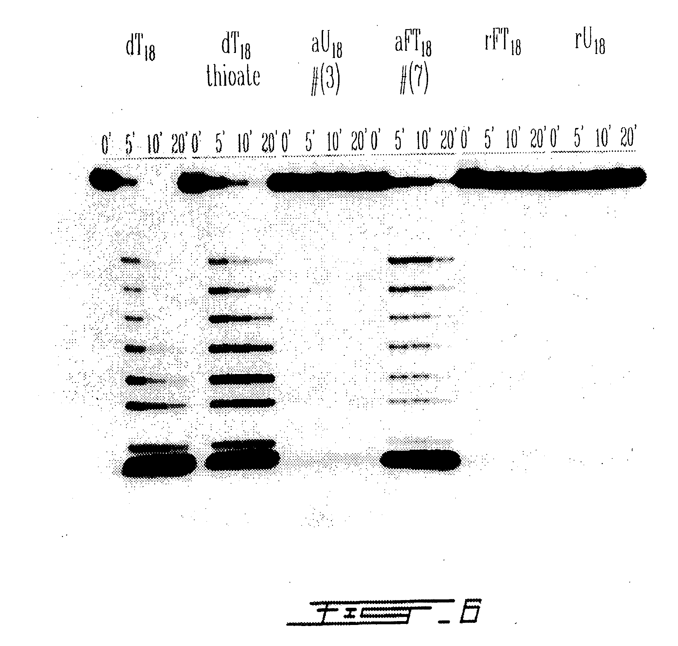

The ability of the above oligonucleotides to elicit RNaseH degradation of target RNA was determined in assays (10 µL final volume) that comprised 5 pmol of 5'-[³²P]- target RNA and 15 pmol of test oligonucleotide in 60 mM Tris-HCl (pH 7.8, 25°C) containing 2mM dithiothreitol, 60 mM KCl, and either 10 mM MgCl₂ or 0.1 mM MnCl₂. Reactions were started by the addition of HIV-RT or E. *coli* RNaseH. Incubations were carried out at 25°C for varying times (generally 20 to 30 minutes). Reactions were quenched by the addition of loading buffer (98% deionized formamide containing 10 mM EDTA and 1mg/mL each of bromophenol blue and xylene cyanol), and heating at 100°C for 5 minutes. The reaction products were resolved by electrophoresis using a 16% polyacrylamide sequencing gel containing 7 M urea, and visualized by autoradiography. The result of such experiments is shown in Fig. 5.

The results show that the oligonucleotide based on 2'-deoxyribose ("DNA" (SEQ ID NO:14)) or β-D-arabinose ("ARA" (SEQ ID NO:1)) are able to form duplexes with target RNA that serve as substrates for the RNase H activity of either HIV-1 RT or *E. coli* RNase H, as indicated by the numerous smaller sized degradation products of the target RNA in Fig. 5. This RNase H degradation was noted with either Mn²⁺ (illustrated) or Mg²⁺ (not shown) as metal. In contrast, oligonucleotides based on D-ribose, either in 3',5'-linkages (3',5'-RNA (SEQ ID NO:15)), or in 2',5'-linkages (2',5'-RNA (SEQ ID NO:16)) were unable to elicit this RNase H degradation of target RNA, even though these test oligonucleotides were competent to form duplexes with the target RNA. Similarly, an oligonucleotide based on β-D-2'-deoxyribose, but of a random base sequence (DNA random) not complementary to the target RNA (and therefore unable to form duplexes), was also unable to elicit RNase H activity.

### EXAMPLES 6

### Induction of Ribonuclease H (RNAseH) Activity by Oligonucleotides possessing 2-Deoxy-2-Fluoro-β-D-Arabinose as Sugar Component

One set of experiments (A) made use of test homopolymeric octadecanucleotides with either thymine (T) or uracil (U) as base component, namely PO-araU₁₈ (SEQ ID NO:3), PO-2'F-araT₁₈ (SEQ ID NO:7), PO-dT₁₈, PS-dT₁₈, PO-2'F-riboT₁₈, PO-riboU₁₈, The target RNA in experiment set A was a synthetic 3',5'-phosphodiester-linked rA₁₈ oligonucleotide, exactly complementary to the sequence of the test oligonucleotides.

Another set of experiments (B) made use of test heteropolymeric octadecanucleotides of the following sequence:
5'-TTA TAT TTT TTC TTT CCC-3' (SEQ ID NO:9) for PO-DNA, PS-DNA and 2'F-ANA oligonucleotides
5'-UUA UAU UUU UUC UUU CCC-3' for ANA (SEQ ID NO:4) and RNA oligonucleotides

The target RNA in experiment set B was a heteropolymeric octadecaribonucleotide exactly complementary to the sequence of the test oligonucleotides.

### Experiments set (A):

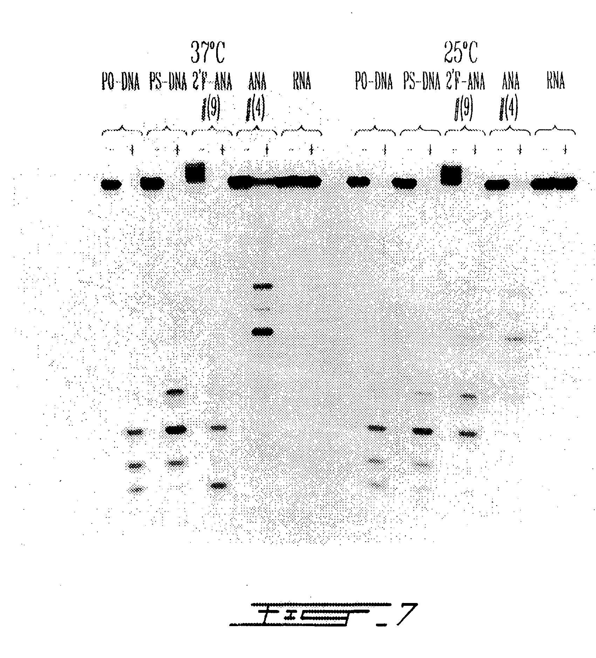

The ability of homopolymeric oligonucleotides with 2'-deoxy-2'-fluoro-β-D-arabinose as sugar component, and other oligonucleotides, to elicit RNaseH degradation of target RNA was determined in assays (10 µL final volume) that comprised 1 pmol of 5'-[³²P]- target RNA and 8 pmol of test oligonucleotide in 60 mM Tris-HCl (pH 7.8, 25°C) containing 2mM dithiothreitol, 60 mM KCl, and 2.5 mM MgCl₂. Reactions were started by the addition of E. *coli* RNaseH. Incubations were at 22°C for 0, 5, 10 and 20 minutes. Reactions were quenched by the addition of loading buffer (98% deionized formamide containing 10 mM EDTA and 1mg/mL each of bromophenol blue and xylene cyanol, and heating at 100°C for 5 minutes. The reaction products were resolved by electrophoresis using a 16% polyacrylamide sequencing gel containing 7 M urea, and visualized by autoradiography. The result of such an experiment is shown in Fig. 6.

Each of the oligonucleotides based on β-D-2'-deoxyribose with phosphodiester bonds (i.e., PO-dT₁₈, abbreviated as "dT₁₈"), β-D-2'-deoxyribose with phosphorothioate bonds (PS-dT₁₈, or "dT₁₈thioate"), 2'-deoxy-2'-fluoro-β-D-arabinose (PO-2'F-araT₁₈ SEQ ID NO:7, or "aFT₁₈"), β-D-ribose (PO-rU₁₈, or "rU₁₈") and 2'-deoxy-2'-fluoro-β-D-ribose (PO-2'F-rT₁₈, or "rFT₁₈") were able to form duplexes with target RNA (rA₁₈). Only duplexes formed with oligonucleotides "dT₁₈", "dT₁₈thioate" or "aFT₁₈" served as substrates for the RNase H activity of either HIV-1 RT or *E. coli* RNase H, as indicated by the numerous smaller sized degradation products of the target RNA in Fig. 6. Duplexes formed with rFT₁₈ or rU₁₈ could not serve as substrates for the RNase H activity of *E. coli* RNase H. Under these conditions an octade-canucleotide based on β-D-arabinosyluracil (PO-araU₁₈ SEQ ID NO:3, or "aU₁₈") was unable to form a duplex with target rA, and accordingly was unable to elicit RNase H activity (these findings are consistent with those reported by Giannaris and Damha, who found that araU₈ was unable to form a duplex with poly rA; Giannaris, P.A.; Damha, M.J., *Can.J.Chem.* 1994, *74*, 909).

### Experiments set (B):

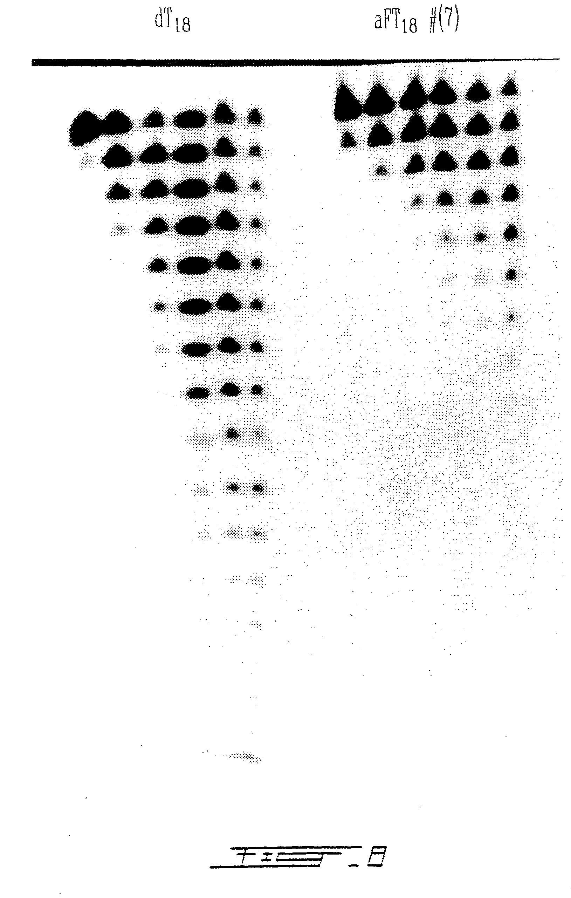

Heteropolymeric octadecanucleotides of the sequence 5'-TTA TAT TTT TTC TTT CCC-3' (SEQ ID NO:9) for PO-DNA, PS-DNA and 2'F-ANA oligonucleotides, and 5'-UUA UAU UUU UUC UUU CCC-3' (SEQ ID NO:4) for ANA and RNA oligonucleotides were annealed to 5'-[³²P]-labeled target RNA exactly complementary to the test AON sequence. The ability of heteropolymeric oligonucleotides with 2'-deoxy-2'-fluoro-β-D-arabinose as sugar component, and other oligonucleotides, to elicit RNaseH degradation of target RNA was determined in assays (50 µL final volume) that comprised 100 nM 5'-[³²P]- target RNA and 500 nM test oligonucleotide in 60 mM Tris-HCl (pH 7.8, 25°C) containing 2mM dithiothreitol, 60 mM KCl, and 2.5 mM MgCl₂. Reactions were started by the addition of E. *coli* RNaseH (final activity 25U/ml). RNaseH digestions were carried out at either 25°C or 37°C. At various times, 10 µL aliquots were removed and quenched by the addition of loading buffer (98% deionized formamide containing 10 mM EDTA and 1mg/mL each of bromophenol blue and xylene cyanol) followed by heating at 100°C for 5 minutes. The reaction products were resolved by electrophoresis using a 16% polyacrylamide sequencing gel containing 7 M urea, and visualized by autoradiography. The result of such an experiment is shown in Fig. 7. The susceptibility of pre-formed oligonucleotide/RNA duplexes to degradation by *E. coli* RNaseH was assessed at 37°C (left panel) and 25°C (Fig. 7, right panel). For each test compound, the lanes correspond to the absence (-) or the presence (+) of added *E. coli* RNaseH.

All heteropolymeric oligonucleotides were able to form duplexes with target RNA (UV melting experiments). Oligonucleotides based on β-D-2'-deoxyribose with phosphodiester bonds (PO-DNA), β-D-2'-deoxyribose with phosphorothioate bonds (PS-DNA), β-D-arabinose (ANA, SEQ ID NO:4) and 2'-deoxy-2'-fluoro-β-D-arabinose (2'F-ANA, SEQ ID NO:9) were able to elicit degradation of the complementary target RNA in the presence of *E. coli* RNaseH, as indicated by the numerous smaller sized degradation products of the target RNA in Fig. 7. Oligonucleotides based on β-D-ribose (RNA) could not elicit RNaseH degradation of the target RNA.

### EXAMPLE 7

### Nuclease Stability of Oligoarabinonucleotides

Thymine octadecanucleotides based on 2'-deoxyribose with phosphodiester bonds (PO-DNA dT₁₈, abbreviated as "dT") and 2'-deoxy-2'-F-β-D-arabinose (2'F-araT₁₈ SEQ ID NO:7, or "aFT₁₈") were compared for stability against degradation by serum nucleases and cellular nucleases. The antisense oligonucleotides were radioactively labeled at the 5'-terminus using [γ-³²P]-ATP and and T4 polynucleotide kinase according to standard protocols (Ausubel, F.M. et al., *Current Protocols in Molecular Biology,* John Wiley & Sons, Inc., 1994).

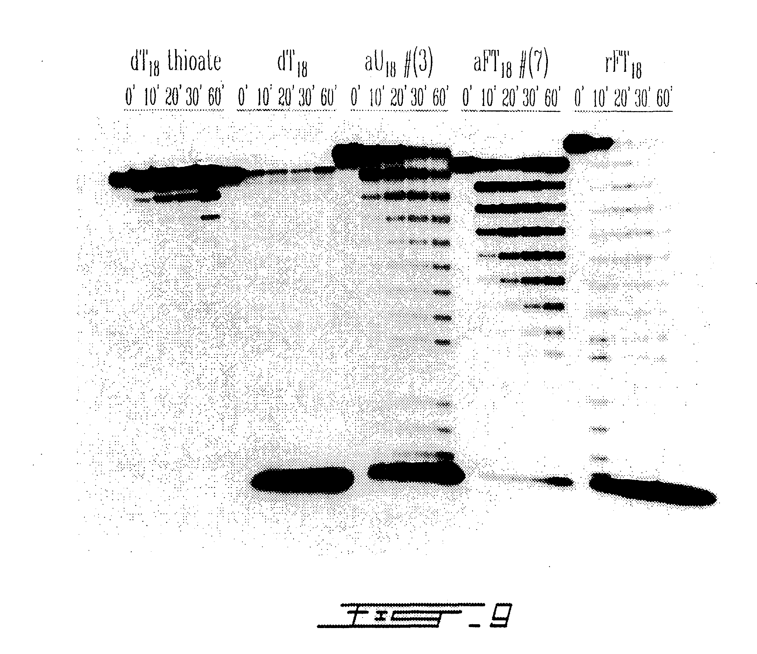

Stability against serum nucleases was assessed by adding 1 pmol of 5'-[³²P]-AON to a reaction assay (10 µL final volume) containing 90% horse serum. Reactions were incubated at 20°C for varying times (0, 5, 10, 15, 20 and 30 min.), then aliquots were removed and diluted with gel loading buffer (98% deionized formamide containing 10 mM EDTA and 1 mg/mL each of bromophenol blue and xylene cyanol), boiled for 5 minutes then resolved by electrophoresis on a 16% polyacrylamide sequencing gel containing 7 M urea. Separated products were visualized by autoradiography. The results of such an experiment is shown in Fig. 8.

The "aFT₁₈" oligonucleotide (SEQ ID NO:7) was substantially more resistant to degradation by serum nucleases, as indicated by the decreased number of smaller molecular size degradation products compared to that noted with normal "dT₁₈" oligonucleotide. Qualitatively similar results were obtained in similar experiments in which human serum was substituted for horse serum (data not presented). Under these same conditions, oligonucleotides based on β-D-2'-deoxyribose with phosphorothioate bonds (PS-dT₁₈) were virtually unaffected by serum nucleases (data not shown), as previously established by many investigators.

Unfractionated mouse liver crude homogenates (prepared by homogenizing mouse livers in an equal volume of 20 mM Tris-HCl (pH 7.9, 20°C) containing 60 mM KCl, 1mM dithiothreitol and 12% (v/v) glycerol) were used as a source of cellular nucleases. Stability against cellular nucleases was assessed by adding 1 pmol of 5'-[³²P]-ODN to a reaction assay (10 µL final volume) containing 90% unfractionated mouse liver crude homogenate. After varying times (0, 10, 20, 30 and 60 min.) of incubation at 20°C, aliquots were removed, diluted with gel loading buffer (98% deionized formamide containing 10 mM EDTA, 1mg/mL each of bromophenol blue and xylene cyanol), boiled for 5 minutes then resolved by electrophoresis on a 16% polyacrylamide sequencing gel containing 7 M urea. Separated products were visualized by autoradiography. The results established that the "aFT₁₈" oligonucleotide was significantly more resistant than the corresponding "dT₁₈" oligonucleotide to degradation by cellular nucleases (data not shown).

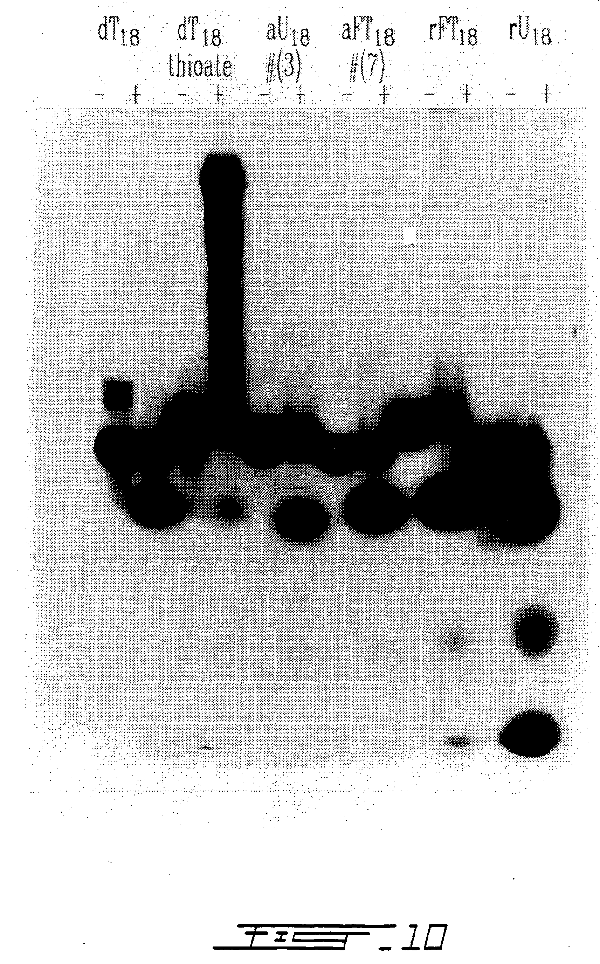

Snake venom phosphodiesterase I is an aggressive enzyme that rapidly degrades single strand nucleic acids. Each of the oligonucleotides based on β-D-2'-deoxyribose with phosphodiester bonds (PO-DNA dT₁₈, abbreviated as "dT"), β-D-2'-deoxyribose with phosphorothioate bonds (PS-DNA dT₁₈, or "dT₁₈ thioate"), β-D-arabinosyluracil (PO-araU₁₈ SEQ ID NO:3, or "aU₁₈"), 2'-deoxy-2'-fluoro-β-D-arabinose (PO-2'-F-araT₁₈ SEQ ID NO:7, or "aFT₁₈") and 2'-deoxy-2'-fluoro-β-D-ribose (PO-2'-F-rT₁₈, or "rFT₁₈") was examined for stability against degradation by snake venom phosphodiesterase I in assays (50 µL final volume) that comprised 100 nM 5'-[³²P]-oligonucleotide in 100 mM Tris-HCl (pH 8.9, 37°C) containing 100 mM NaCl and 10 mM MgCl₂. Reactions were started by the addition of snake venom phosphodiesterase I (final activity 0.4U/ml). Digestions were carried out at or 37°C. At various times, 10 µl aliquots were removed and quenched by the addition of loading buffer (98% deionized formamide containing 10 mM EDTA and 1mg/mL each of bromophenol blue and xylene cyanol) followed by heating at 100°C for 5 minutes. The reaction products were resolved by electrophoresis using a 16% polyacrylamide sequencing gel containing 7 M urea, and visualized by autoradiography. The results of such an experiment is shown in Fig. 9. They established that the order of nuclease stability is "dT₁₈ thioate" > "aU₁₈" ≈ "aFT₁₈" » "rFT₁₈" > "dT₁₈".

### EXAMPLE 8

### Nonspecific Interaction of Oligoarabinonucleotides with Cellular Proteins

The ability of thymine octadecanucleotides based on β-D-2'-deoxyribose with phosphodiester bonds (PO-DNA dT₁₈, abbreviated as "dT₁₈"), β-D-2'-deoxyribose with phosphorothioate bonds (PS-DNA dT₁₈, or "dT₁₈ thioate"), β-D-arabinosyluracil (PO-araU₁₈ SEQ ID NO:3, or "aU₁₈"), 2'-deoxy-2'-fluoro-β-D-arabinose (PO-2'-F-araT₁₈ SEQ ID NO:7, or "aFT₁₈"), and 2'-deoxy-2'-fluoro-β-D-ribose (PO-2'-F-rT₁₈, or "rFT₁₈") to bind nonspecifically to proteins in a Hela cell crude extract was analyzed by a gel shift assay procedure. The antisense oligonucleotides were radioactively labeled at the 5'-terminus using [γ-³²P] -ATP and and T4 polynucleotide kinase according to standard protocols (Ausubel, F.M. et al., *Current Protocols in Molecular Biology,* John Wiley & Sons, Inc., 1994). Hela cell crude extracts were prepared by homogenizing cells in buffer (20 mM Tris-HCl (pH 7.9, 20°C) containing 60 mM KCl, 1mM dithiothreitol and 12% (v/v) glycerol), followed by centrifugation to remove membrane particles and cell debris.

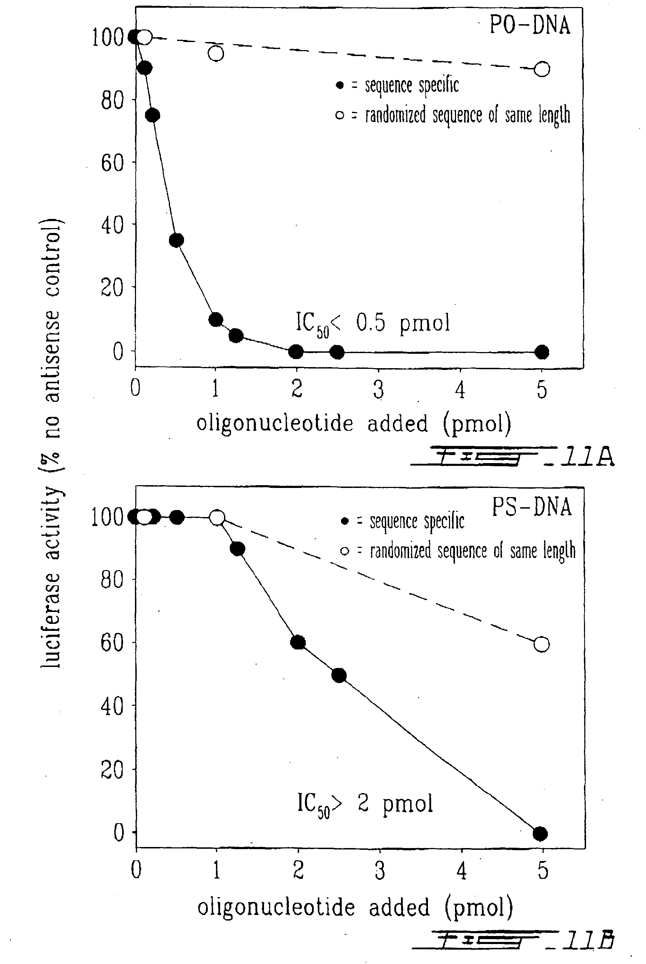

The binding experiments comprised 1 pmol of 5'-³²P-labeled AON and 5 µg Hela cell extract protein in a total volume of 20 µl comprising 10 mM Tris-HCl (pH 7.5, 25°C) containing 100 mM KCl, 1 mM MgCl₂, 1 mM EDTA and 5 % glycerol. AON and Hela cell proteins were incubated for 10 min at 25°C, then electrophoresed on 6% non-denaturing gels. Following completion of the electrophoresis, the gels were dried and the positions of the free and protein-bound oligonucleotides visualized by autoradiography. The results of such an experiment are shown in Fig. 10.

Essentially all of the phosphorothioate "dT₁₈ thioate" was bound to the extract proteins, as indicated by the "smear" of radioactive material throughout the extent of electrophoresis. None of the other antisense oligonucleotides showed any appreciable interaction with Hela cell proteins under the same conditions.

### EXAMPLE 9

### Antisense oligonucleotide inhibition of specific gene expression

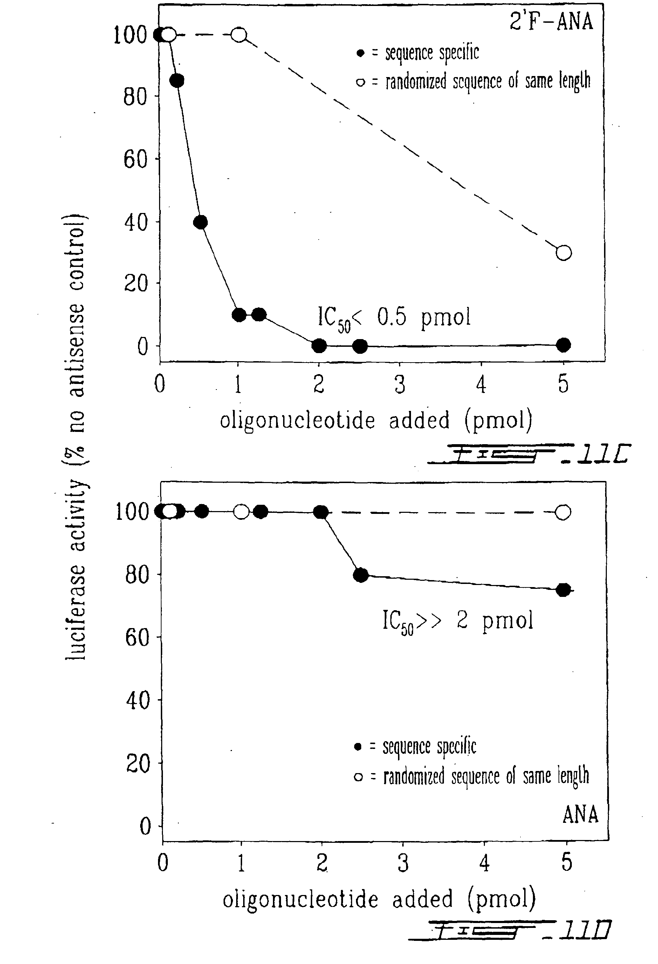

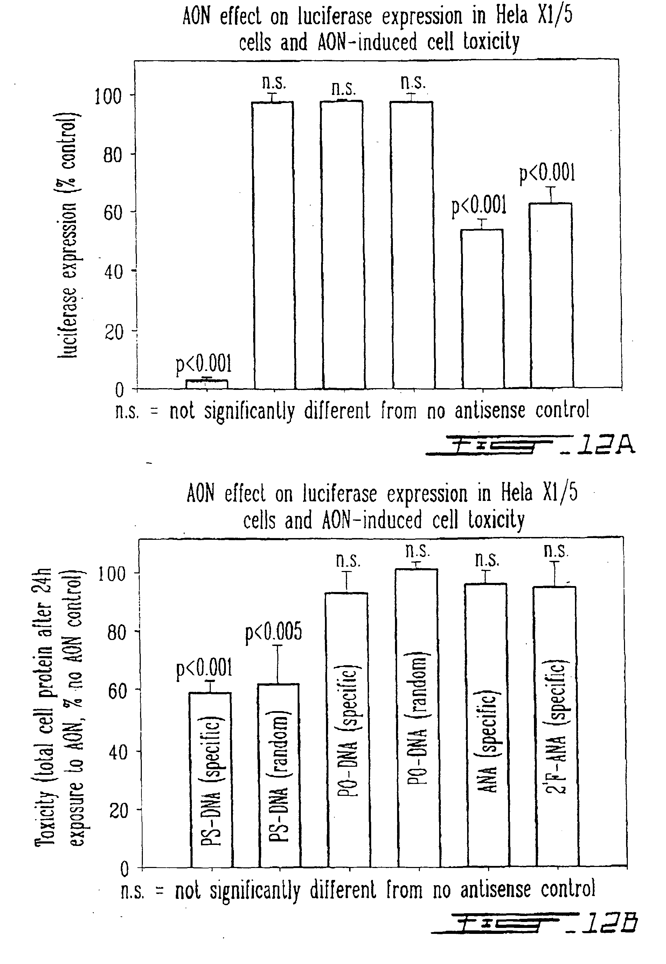

Antisense oligonucleotides have the potential to inhibit expression of virtually any gene, based on the specific base sequence of the chosen target mRNA. We examined the ability of antisense oligonucleotides based on β-D-arabinose and 2'-deoxy-2'-fluoro-β-D-arabinose to interfere with the expression of a well-characterized marker model, namely expression of the enzyme luciferase, in both *in vitro* cell-free translation experiments (Fig. 11), and in cells stably transfected with the luciferase gene (Fig. 12).

The ability of oligonucleotides complementary to a specific region of mRNA coding for luciferase was tested for inhibition of luciferase protein expression in an *in vitro* protein translation system. The specific antisense oligonucleotide sequences were 5'-ATA TCC TTG TCG TAT CCC-3' (SEQ ID NO:10) for 2'F-ANA, ANA (SEQ ID NO:5) and the corresponding PO-DNA and PS-DNA strands, which are complementary to bases 1511-1528 of the coding region of the luciferase gene (M. Gossen, H. Bujard 1992, *Proc. Natl. Acad. Sci. USA.* 89, 5547-5551; (M. Gossen, H. Bujard 1992, *Proc. Natl. Acad. Sci. USA.* 89, 5547-5551; W.M. Flanagan *et al.* 1996, *Nucl. Acids Res.* 24, 2936-2941). As a control, randomized oligonucleotide sequences (5'-TAA TCC CTA TCG TCG CTT-3' (SEQ ID NO:17) for 2'F-ANA, ANA, PO-DNA and PS-DNA were used; these are of the same base composition as the specific AON sequence, but have no complementarity to any portion of the luciferase gene. Translation reaction assays (15 µl total volume) comprised 0.15 pmol luciferase mRNA, 10 µl commercial rabbit reticulocyte lysate supplemented with complete amino acids mixture and excess single strand RNA ribonuclease inhibitor. To this mixture was added varying amounts (0.1 - 5 pmol) of specific or random oligonucleotide, followed by addition of *E. coli* RNaseH (20U/ml final concentration). Translation reactions were carried out for 60 min at 37°C, then the amount of full-length active luciferase produced was assayed by luminometry (W.M. Flanagan et al 1996, *Nucl. Acids* Res. 24, 2936-2941). The results of such an experiment are shown in Fig. 11. Panel (A) shows the inhibitory activity of oligonucleotides based on β-D-2'-deoxyribose with phosphodiester bonds (PO-DNA). Panel (B) shows the inhibitory activity of oligonucleotides based on β-D-2'-deoxyribose with phosphorothioate bonds (PS-DNA) Panel (C) shows the inhibitory activity of oligonucleotides based on 2'-deoxy-2'-fluoro-β-D-arabinose with phosphodiester bonds (2'F-ANA SEQ ID NO:10); Panel (D) shows the inhibitory activity of oligonucleotides based on β-D-arabinose with phosphodiester bonds (ANA, SEQ ID NO:5).

The results (Fig. 11) show that 2'F-ANA and PO-DNA oligonucleotides are potent and specific inhibitors of luciferase gene expression in the *in vitro* protein expression model, at low AON:mRNA ratios. In these experiments, the *in vitro* inhibitory potency (IC₅₀) of oligonucleotides based on 2'-deoxy-2'-fluoro-β-D-arabinose with phosphodiester bonds (2'F-ANA SEQ ID NO:10) is at least four-fold greater than that of the same-sequence oligonucleotide based on β-D-2'-deoxyribose with phosphorothioate bonds (PS-DNA).

Hela X1/5 cells stably express the luciferase gene (W.M. Flanagan et al 1996, *Nucl. Acids Res.* 24, 2936-2941). Cells were treated with Lipofectin and oligonucleotide (1 µM final concentration) for 24h, then cells were harvested and cell extracts were assayed for luciferase activity (Fig. 12, panel A) and toxicity (residual cell protein, Fig. 12, panel B). The chemical character of the oligonucleotides is indicated on Fig. 12. Specific sequences used are as those described above, namely, 2'F-ANA (SEQ ID NO:10), ANA (SEQ ID NO:5) and their corresponding PO-DNA and PS-DNA sequences. Random sequences were 2'F-ANA (SEQ ID NO:17, ANA and corresponding randomized PO-DNA and PS-DNA sequences..

X1/5 Hela cells were plated in 96-well plates and allowed to grow, in DMEM/10% FBS, to 80% confluence, as assessed by microscopy. The medium was removed, and the cells washed several times with phosphate-buffered saline. The cells were overlaid with serum-free DMEM medium containing 20 µg/ml Lipofectin, then a small volume aliquot of concentrated test AON stock solution was added with mixing to ensure good distribution. After 24h incubation, the Hela cells were harvested, homogenized and assayed for luciferase.

Panel A of Fig. 12 shows that PS-DNA (sequence specific) completely eliminated intracellular luciferase gene expression. The 2'F-ANA (SEQ ID NO:10)and ANA (SEQ ID NO:5) sequence specific oligomers were also potent inhibitors decreasing intracellular luciferase gene expression by about 40-50%. These data were significantly different from the PO-DNA control as indicated in the Panel A (statistical significance is indicated as well). Panel B shows that neither 2'F-ANA or ANA were toxic to Hela cells as determined by effect on cell number (i.e., residual cell protein after 24-h exposure). In contrast, PS-DNA (both random and specific sequences) exhibited significant toxicity reducing cell number by ca. 40% after 24-h exposure.

## Claims

1. Therapeutic composition for selectively preventing or modulating gene expression in a sequence-specific manner in a host, wherein said composition comprises an effective amount of at least one oligonucleotide, wherein said oligonucleotide is a uniformly sugar-modified oligonucleotide, based on 2'-deoxy 2'-fluoro-β-D-arabinonucleotides, and wherein said oligonucleotide is capable of hybridizing to complementary mRNA and inducing (RNase H)-mediated cleavage thereof

2. Therapeutic composition according to claim 1, wherein said oligonucleotide has the formula: 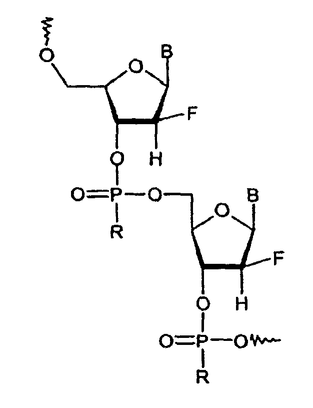 wherein:
B is selected from the group consisting of adenine, guanine, uracil, thymine, cytosine, inosine, and 5-methylcytosine;
R at the internucleotide phosphate linkage is selected from the group consisting of Hydroxyl oxygen, Thiol, methyl, amino, alkylamino, dialkylamino (the alkyl group having one to about 20 carbon atoms), methoxy, and ethoxy.

3. Therapeutic composition according to claim 2, wherein R at the internucleotide phosphate linkage is Hydroxyl.

## Patentansprüche

1. Therapeutische Zusammensetzung zur selektiven Verhütung oder Modulation der Genexpression in einer sequenz-spezifischen Art in einem Wirt, wobei die Zusammensetzung eine effektive Menge mindestens eines Oligonukleotids umfasst, wobei das Oligonukleotid ein einheitlich zucker-modifiziertes Oligonukleotid ist, basierend auf 2'-Desoxy-2'-fluor-β-D-arabinonukleotiden, und wobei das Oligonukleotid zur Hybridisierung mit komplementärer mRNS und zur Induzierung deren durch (RNase H)-vermittelter Spaltung imstande ist.

2. Therapeutische Zusammensetzung gemäß Anspruch 1, wobei das Oligonukleotid die Formel 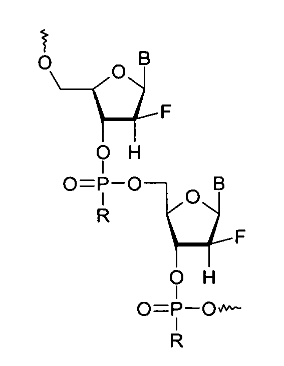 aufweist, wobei:
B ausgewählt ist aus der Gruppe bestehend aus Adenin, Guanin, Uracil, Thymin, Cytosin, Inosin und 5-Methylcytosin;
R an der internukleotid-phosphat-Verbindung ausgewählt ist aus der Gruppe bestehend aus Hydroxy-, Thiol-, Methyl-, Amino-, Alkylamino-, Dialkylamino- (die Alkylgruppe weist ein bis etwa 20 Kohlenstoffatome auf), Methoxy- und Ethoxy-Gruppe.

3. Therapeutische Zusammensetzung gemäß Anspruch 2, wobei R an der internukleotid-phosphat-Verbindung eine Hydroxy-Gruppe ist.

## Revendications

1. Composition thérapeutique destinée à éviter ou à moduler sélectivement la transcription d'un gène d'une manière spécifique de la séquence chez un hôte, dans laquelle ladite composition comprend une quantité efficace d'au moins un oligonucléotide, dans laquelle ledit oligonucléotide est un oligonucléotide uniformément modifié par un sucre, basé sur les 2'-déoxy-2'-fluoro-β-D-arabinonucléotides, et dans laquelle ledit oligonucléotide est capable de s'hybrider à un ARNm complémentaire et à induire un clivage de celui-ci sous la médiation de la RNase H.

2. Composition thérapeutique selon la revendication 1, dans laquelle ledit oligonucléotide a la formule : 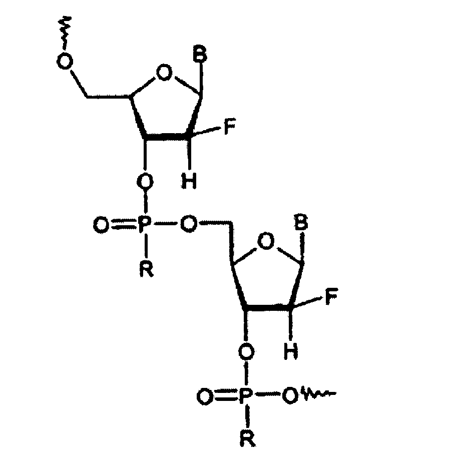 dans laquelle :
B est choisi dans le groupe consistant en une adénine, une guanine, un uracile, une thymine, une cytosine, une inosine et une 5-méthylcytosine ;
R au niveau de la liaison phosphate inter-nucléotides est choisi dans le groupe consistant en un hydroxyle, un thiol, un méthyle, un amino, un alkylamino, un dialkylamino (le groupe alkyle ayant de un à environ 20 atomes de carbone), un méthoxy et un éthoxy.

3. Composition thérapeutique selon la revendication 2, dans laquelle R au niveau de la liaison phosphate inter-nucléotides est un hydroxyle.
